(19) Europäisches Patentamt
European Patent Office
Office européen des brevets

(11) EP 2 490 670 B1

(12) **EUROPEAN PATENT SPECIFICATION**

(45) Date of publication and mention of the grant of the patent:
**17.04.2019 Bulletin 2019/16**

(21) Application number: **10781721.5**

(22) Date of filing: **21.10.2010**

(51) Int Cl.:
***A61K 9/12*** *(2006.01)* ***A61K 9/14*** *(2006.01)*
***A61K 9/16*** *(2006.01)* ***A61K 9/72*** *(2006.01)*

(86) International application number:
**PCT/GB2010/001955**

(87) International publication number:
**WO 2011/048379 (28.04.2011 Gazette 2011/17)**

(54) **COMPOSITION FOR INHALATION**

ZUSAMMENSETZUNG ZUM INHALIEREN

COMPOSITION POUR INHALATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2009 GB 0918450**

(43) Date of publication of application:
**29.08.2012 Bulletin 2012/35**

(73) Proprietor: **Innovata Limited**
**Chippenham**
**Wiltshire SN14 6FH (GB)**

(72) Inventor: **MARTYN, Glen**
**Chippenham**
**Wiltshire SN14 6FH (GB)**

(74) Representative: **Potter Clarkson**
**The Belgrave Centre**
**Talbot Street**
**Nottingham NG1 5GG (GB)**

(56) References cited:
**WO-A1-89/06976** **WO-A2-98/16205**
**WO-A2-03/000202** **WO-A2-2007/042822**
**WO-A2-2010/111680**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 490 670 B1

## Description

The invention relates to compositions suitable for administration of bioactive materials, methods of forming the compositions and methods for reducing agglomeration of particles and/or particle blends and/or for increasing the stability and/or dispersibility of particles and/or particle blends and/or powders and/or microcapsules to moisture and/or heat, the use of efflorescent salts in a dry powder inhaler composition to improve, for example, the filling, flow and dispersion of said powder. Other advantages of the invention will be apparent from the following description.

The flow and dispersion of aerosols from dry powder inhalers (DPIs) can be modified by the presence of a variety of components. Lucas describes that fine particle excipients can be used to improve the performance of carrier-based protein dry powder aerosols [Pharm Res. 1998 Apr;15(4):562-9]. Islam discloses that dispersion behaviour is associated with the presence of fine adhered particles associated with the carrier powder [http://www3.interscience.wiley.com/cgi-bin/abstract/107613204/ABSTRACT]. Zeng [J Pharm Sci. 2001 Sep;90(9):1424-34.] showed how the effects of surface asperities or cavities of lactose were offset by introducing a small amount (5% w/w) of smaller-sized lactose (5-10 microns) to the powder formulations. The fine lactose increased the FPF and dispersibility of albuterol and it was found that the inclusion of recrystallized needle lactose (5-15 microns) was superior to micronized lactose in improving the aerosolization of albuterol.

EP 1131059 - Jago - describes the use of magnesium stearate for stabilization of dry powder inhalation formulations to improve resistance to moisture. One of the claimed features of the dry powder is that a high fine particle dosage or fine particle fraction can be maintained also under relatively extreme temperature and humidity conditions.

US 6528096 - Chiesi - discloses powdery pharmaceutical compositions including an active ingredient and carrier particles containing only a small amount of lubricant, 0.1-0.5% by weight, which are used to prepare dry powder inhalers in order to increase the fine particle dose. The list of desired lubricants includes magnesium stearate and the like.

WO 962348 - CDD Ltd - discloses powders for use in dry powder inhalers which comprise active particles, carrier particles, and an additive material (such as leucine) which promotes the release of active particles on actuation of the inhaler.

All of the above documents describe hydrophobic additives for improving the flow and dispersion as well as reducing the moisture sensitivity of dry powder inhalation formulations. The only hydrophilic additives disclosed are amino acids and their salts. Only magnesium stearate is present in two marketed DPI formulations - Foradil® Certihaler® (Novartis) and Chiesi's Pulvinal®- and, being a soap, it is likely to have taste issues.

WO 96/03978 discloses solid delivery systems for the controlled release of molecules incorporated therein.

WO 90/11756 describes slow release vitreous systems comprising a water soluble glass having a softening point of less than 320°C wherein the water soluble glass has admixed therein an active agent.

WO 95/24183 discloses methods and compositions for pulmonary delivery of insulin.

WO 98/16205 describes stable glassy state powder compositions. The compositions generally comprise polyols. This document is not concerned with how to improve stability at high temperature and high humidity and all of the examples include sodium citrate as a metal salt generally with glycine and mannitol.

EP 0563455 discloses particulate solid products containing a lump forming inhibitor which is calcium lactate. This document describes large particles having a size of from 1 to 5cm which would not be suitable for inhalation.

WO 2007/042822 describes a unit dose dry powder inhaler and a dry powder formulation for inhalation which comprises a desiccant and a bioactive material. WO 2007/042822 does not disclose glassy matrices formed from a metal ion salt which do not comprise stabilising polyols.

WO 99/47174 discloses drying a compound which is subject to deactivation on drying in the presence of one or more monosaccharide sugar alcohols and at least one additive which is a glass-former or a glass-formation facilitator.

WO 89/06976 describes methods of drying macromolecules which comprises formulating in the presence of one or more efflorescent alkali metal, ammonium or alkaline earth metal salts and then drying.

WO 00/18259 discloses the incorporation of one or more anhydrous compounds into a nutritional or pharmaceutical composition in an amount capable of sequestering any water which may be released from one or more water containing components.

WO 2004/060343 describes antibody-containing particles and compositions for reconstitution with a suitable diluent.

WO 03/079993 discloses hGH (human growth hormone) formulations for pulmonary administration.

None of these documents disclose the co-drying of a bioactive material with a glass forming metal ion salt, such as an efflorescent salt, for example in the absence of a stabilising polyol or other glass former, to provide a dry powder for pulmonary administration.

There remains a need for improved formulations comprising inert additives to produce more efficient and stable DPI formulations.

There also remains a need for compositions, particularly compositions suitable for inhalation, which are stable under conditions of high relative humidity and/or heat, which maintain their particle size distribution without the need for

complicated storage requirements and which remain free-flowing during filling and prior to administration.

The subject matter of the invention is set out in the appended claims.

As used herein the term "stability" can mean both or either of the physical stability of the composition and/or the physical and/or chemical stability of the composition and/or the bioactive material in the composition. For example in relation to a particulate composition physical stability preferably means the tendency of the particles to agglomerate and/or clump thus reducing dispersibility in an inhaler. The compositions of the invention may have improved physical stability compared to compositions which do not comprise the metal ion salt and/or which have not been prepared according to the methods of the invention.

For example, where the bioactive material is labile, such as, for example, a protein or enzyme which can be denatured by heat and/or solvents, or a small molecule which is labile to air and/or water, the compositions of the invention, such as glassy powders, can maintain the physical and/or chemical stability of the bioactive material. The chemical stability may refer to maintaining the activity of the bioactive material to a particular level, such as at least about 70, 80, 90, 95 or 99% of an initial level before incorporation into the composition or an amount of aggregation less than about 10%, 5%, 2%, 1%, 0.5% or 0.1 % compared to the bioactive material before incorporation into the composition.

By the terms "powder" or "particulate composition" it is intended to mean a collection or mass of particles. The powder or particulate composition is suitable for inhalation or pulmonary administration. The particles may have any shape and they may be, for example, hollow, solid, porous or non-porous. For example, the particles may be in the form of needles, fibers, discs or spheres The particles may, for example, be microparticles such as microneedles or microfibers, or microspheres such as described in WO 96/03978. The particles have a median geometric diameter less than 10 μm. For example, the particles may have a median geometric diameter of about 0.1 to 5 μm or 1 to 2 μm.

Figure 1 shows a DSC trace for a spray-dried trehalose and insulin powder.

Figure 2. shows a DSC trace for melt quench calcium lactate.

Figure 3 shows a DSC trace for a spray-dried calcium lactate and insulin powder.

Figures 4 and 5 show the stability of calcium lactate and insulin powders.

The invention can be considered to relate, at least in part, to the finding that the metal ion salts in an amorphous and/or anhydrous and/or lower hydrate form, particularly without the use of additional glass-formers, can provide unexpected stabilisation of, for example, powders comprising relatively small particles, to humidity and/or heat, for example in terms of reduced agglomeration and/or increased dispersibility compared to formulations which do not contain the metal ion salt. In addition, the use of a metal ion salt in an amorphous and/or anhydrous and/or lower hydrate form can also provide free-flowing powders over a range of challenging environmental conditions. Without wishing to be bound by theory, it is believed that the surprisingly high glass transition temperatures and/or high $AC_P$ of metal ion salts having a low molecular weight and/or the degree of hydration of the salt may contribute to improved powder stabilisation. For example, the water sequestering capacity of an anhydrous or lower hydrate form of a metal ion salt may serve to improve and/or maintain dispersibility and/or reduce agglomeration when co-formulated with a bioactive in compositions for inhalation.

By "amorphous form" it is intended to mean that the metal ion salt is not crystalline. For example, the metal ion salt may be in the form of a glass, which is amorphous. The metal ion salts used in the invention are glass-forming salts and are in the form of glasses.

The terms "glass", or "glassy state" or "glassy matrix" as used herein refers to a liquid that has lost its ability to flow i.e. it is a liquid with a very high viscosity, wherein the viscosity preferably ranges from $10^{10}$ to $10^{14}$ Pascal seconds. It can be viewed as a metastable amorphous system in which the molecules have vibrational motion and reduced rotational motion but have very slow translational motion when compared to the liquid state. As a metastable state, it is stable for long periods of time when stored well below the glass transition temperature.

The process used to obtain a glassy matrix in the invention is generally via solvent evaporation technique on a composition, such as, for example, spray-drying, although other processes can produce a glassy matrix with an acceptable Tg, such as for example, freeze-drying followed by micronization. The composition to be dried may be in the form of, for example, a solution, dispersion, suspension or emulsion.

The bioactive material can also be dried from a solution of the metal ion salt and the bioactive material to form a metal ion salt carrier glass containing homogeneously distributed bioactive material in solid solution in the glass. These glasses can then be milled and/or micronized to give microparticles of homogeneous defined sizes. Such solid solutions can provide a quick release of the bioactive upon administration and thereby improve absorption and onset of action.

A particularly suitable technique is spray-freeze-drying technology. The process of spray-freeze-drying involves the atomisation of a solution or dispersion of the metal ion salt and/or bioactive material, and then directing the resulting

droplets into a liquified gas, typically liquid nitrogen, or a cryogenic surface. The droplets freeze on contact and may then be dried using a freeze-drying step to remove residual moisture. The resulting microparticles comprise a bioactive material dispersed within the metal ion salt. Prior to microparticle formation, the bioactive material may be in solution or present as a dispersion of microparticles or nanoparticles (with an optional stabiliser) in the feedstock.

The apparatus and process conditions used to produce the initial droplets will be apparent to the skilled person. Feed concentrations, pump rates, atomisation pressures and nozzle types can all be selected based on conventional process conditions, and then optimised according to feedstock concentration and viscosity. The size of the microparticles will be determined in part by the atomisation used in the spray-freeze-drying process. The atomisation/spraying stage may make use of a conventional atomisation process, e.g. pressure or two fluid nozzles, or may utilise an ultrasonic atomisation process (Maa et a/., Pharmaceutical Research, 1999; 16(2)).

The most preferred method of production of powders or particles of the invention is via spray-drying as fully described in, inter alia, WO 92/18164 and WO 96/15814. Microparticles may be spray- dried but modified by the inclusion of a blowing agent, in the feedstock for spray-drying. The blowing agent is a volatile substance which releases a gas or gases during the spray-drying process. Blowing agents can be used in the present invention to produce lower density hollow microcapsules. Suitable blowing agents include ammonium acetate, ammonium hydroxide, ammonium carbonate, ammonium bicarbonate, acetic acid, formic acid and hydrochloric acid. The pH at which these blowing agents are used may vary; this implies that compounds with pH-dependent solubilities can be spray-dried with the addition of a suitable blowing agent. By way of example, the blowing agent used may be ammonium carbonate which releases ammonia, carbon dioxide and water vapour. During spray-drying, these three gases expand in the atomised droplets, causing the droplet to increase in size, to produce larger, thinner-walled microcapsules. Products of the invention may have various characteristics, depending on the conditions of their preparation. Their bulk density may be 0.01 to 0.15 g/cm$^3$ more preferably 0.02 to 0.1 g/cm$^3$, but most preferably 0.04 to 0.8 g/cm$^3$.

Tapped bulk density, or tapped or tap density, is the maximum packing density of a powder (or blend of powders) achieved under the influence of well-defined externally applied forces. The minimum packed volume thus achieved depends on a number of factors including particle size distribution, true density, particle shape and cohesiveness due to surface forces including moisture.

In one embodiment, the particles or microparticles of the invention, have a tap density of less than or equal to about 0.4 g/cm$^3$ or 0.3 g/cm$^3$, for example less than about 0.25 g/cm$^3$, such as less than about 0.2 g/cm$^3$. For example, in one embodiment, the particles or microparticles of the invention have a tap density of from about 0.02 to 0.2 g/cm$^3$, from 0.05 to 0.15 g/cm$^3$, or from 0.07 to 0.12 g/cm$^3$, such as from about 0.08 to 0.10 g/cm$^3$.

Tap density can be measured by using instruments known to those skilled in the art such as the Dual Platform Microprocessor Controlled Tap Density Tester (Vankel Technology, Cary, NC) or a GeoPyc™ instrument (Micrometrics Instrument Corp., Norcross, GA 30093). Tap density can be determined using the method of USP Bulk Density and Tapped Density, United States Pharmacopoeia convention, Rockville, MD, 10$^{th}$ Supplement, 4950-4951, 1999. Preferably, the tap density is measured using a Tap Density Volumeter, Copley.

The bulk density of the microparticles may, in any of the embodiments herein, be less than or equal to about 0.25 or 0.2 g/cm$^3$, such as less than about 0.15 g/cm$^3$. In one embodiment, the bulk density is from about 0.02 to 0.15, 0.2 or 0.25 g/cm$^3$, or from about 0.05 to 0.12 g/cm$^3$.

In one embodiment of the invention, the difference between the tap density and the bulk particle density of the microparticles is less than about 0.07 g/cm$^3$ or less than about 0.05 g/cm$^3$, such as less than 0.03 g/cm$^3$, for example from about 0 to 0.05 g/cm$^3$ or from about 0.01 to 0.03 g/cm$^3$.

A glassy foam may be prepared using the methods described in WO 96/40077.

The composition of the invention in one embodiment comprises a carrier material which is not a metal ion salt. Suitable carrier materials include, for example, carbohydrates such as lactose or glucose.

The amount of water in the composition may be measured by, for example, TGA or Karl Fischer titration. The composition of the invention may have a moisture or water content of less than about 10 wt.%, such as less than about 5, 4, 3, 2 or 1 wt.% based on the total weight of the composition. Typical moisture contents are from about 0.5 to 4 wt.% or 1 to 3 wt.% based on the total weight of the composition.

The metal ion salt used in any of the embodiments herein may have a moisture or water content, such as measured by TGA, of, for example, from about 0 to about 25 wt.%, such as from about 3 or 4 to about 23 wt.%, from about 5 to about 20 wt.%, from about 7 to about 15 wt.%, or from about 8 to about 10 wt.% based on the total weight of the salt. In one embodiment, the metal ion salt has a moisture or water content of less than about 10 wt.%, such as less than about 5, 4, 3, 2 or 1 wt.% based on the total weight of the salt.

By "substantially-free", it is intended to mean that other glass-forming materials are present in an amount of less than 1 or 0.1wt.% based on the weight of the composition, such as less than 0.01 wt.%, less than 0.001 wt.%, less than 0.0001 wt.%, such as 0 wt.%. Examples of glass-forming materials which may be excluded include, for example, carbohydrates, such as monosaccharide alcohols, for example, mannitol, disaccharides and trisaccharides, such as trehalose and raffinose. Amino acids, such as glycine are also preferably absent from the compositions of the invention.

For example, in one embodiment compositions of the invention do not comprise polyols, such as, for example, monosaccharide alcohols, mannitol, sucrose, trehalose and glycine.

In one embodiment, the metal ion salt is the sole glass-forming agent in the composition or is the sole glass-forming additive i.e. added to the bioactive material.

The metal ion salt is selected from calcium lactate, calcium lactate citrate and calcium lactate gluconate. Such materials can be obtained commercially from, for example, PURAC (Illinois, USA) under the trade name PURACAL®.

The compositions of the invention may include one or more metal ion salts or combinations of different metal ion salts as a glass-forming material but in general a single metal ion salt is preferred.

In one embodiment, the powder of the invention comprises particles which may be amorphous, or crystalline, or mixtures thereof. Typically, at least 50 percent by weight of the particles are amorphous in form, wherein crystalline forms make up less than 50 percent by weight of the total weight of the particles, regardless of the nature of individual particles. Preferably, at least 75 percent by weight of the particles are amorphous in form. More preferably, at least 90 percent by weight of the particles are amorphous in form, such as at least 95 wt.% or 99 wt.% based on the total weight of the powder.

The term "efflorescent' is used herein to refer to salts which lose water under ambient humidity, e.g. those salts having a vapour pressure of water of crystallization of at least 15 mm Hg (2000 Pa). An example of an efflorescent salt is calcium lactate hydrate.

The metal ion salt may have no water of crystallization associated with the salt, i.e., be an anhydrate. For example, the metal ion salt may have a moisture content, such as measured by TGA or Karl Fischer, of from about 0.1 or 1 to about 10 wt.% or from about 0.2 or 2 to 5 wt.%.

It is preferred however, that the metal ion salt includes water of crystallization, or is a hydrate at least initially. The number of molecules of water associated with the metal cation and anion stoichiometric formula, [Cation]x[Anion]y. Z $H_20$ (where x and y are integers determined by the valency of the cation and anion and Z is an integer which represents the degree of hydration or number of molecules of water), or water of crystallization, may be expressed in terms of hydrate. For example, with one molecule (or mole) of water per stoichimetric or molar formula, the salt is termed a hydrate, two units of water, a dihydrate and so on.

In one embodiment of the invention, the metal ion salt is or has been treated so as to reduce the level or degree of hydration of the initial metal ion salt. For example, the invention preferably uses a metal ion salt hydrate as a starting material, such as at least a trihydrate or at least a pentahydrate, and this is then subjected to a process which reduces the degree of hydration, such as, for example, by spray-drying. The bioactive material may be present or absent when the process is carried out, but is preferably present. For example, the starting or raw material metal ion salt before processing may have a degree of hydration of at least three (trihydrate) and this is or has been treated so as to reduce the degree of hydration to less than three. The metal ion salt may therefore be a treated metal ion salt.

In one embodiment, the initial or treated metal ion salt is an anhydrate, monohydrate, dihydrate, trihydrate, tetrahydrate, pentahydrate, hexahydrate, heptahydrate, octahydrate, nonahydrate, decahydrate or dodecahydrate. Preferably, the initial or treated metal ion salt is at least a trihydrate, more preferably at least a pentahydrate.

In any of the aspects or embodiments herein, the metal ion salt is preferably pharmaceutically or physiologically acceptable.

The salt is preferably pharmaceutically or physiologically acceptable and/or has been treated so as to reduce the degree of hydration.

Typically, the anhydrous and/or lower hydrate form of a metal ion salt, such as described herein, is formed from or derived from a metal ion hydrate which is or has been treated so as to reduce the level or degree of hydration. The metal ion hydrate which is the starting material may be a monohydrate, dihydrate, trihydrate, tetrahydrate, pentahydrate, hexahydrate, heptahydrate, octahydrate, nonahydrate, decahydrate or dodecahydrate. Preferably, the metal ion salt starting material is at least a trihydrate, more preferably at least a pentahydrate. For example, the metal ion salt may be calcium lactate pentahydrate which is treated so as to reduce the degree of hydration to about zero i.e., the calcium lactate is substantially anhydrous.

The term "lower hydrate" as used herein is intended to mean that the degree of hydration is less than for the initial or starting or raw material metal ion salt.

The metal ion salt is present in the composition in an amount of less than or equal to 30, 20 or 10 wt.% based on the total weight of the composition, such as less than or equal to about 5 wt.%, 2 wt.% or 1 wt.%, for example from 1 to 10 wt.%, 2 to 8 wt.% or 3 to 6 wt.%. In one embodiment, the metal ion salt may be present in trace amounts in the composition.

It is preferable for only small amounts of additive material to reach the lower lung, and it is also highly preferable for the additive material to be a material which may be safely inhaled into the lower lung where it may be absorbed into the blood stream or be removed from the lung (e.g. by the mucocilliary escalator). The metal ion salts according to the invention are particularly suited for use with hygroscopic and other moisture sensitive agents, e.g. those prone to hydrolysis.

Administration of the compositions of the invention may be by oral and/or nasal inhalation. The compositions of the invention may be in a solid dose form selected from, for example, fibers, spheres, tablets, discs, particles and needles of relatively homogeneous size distribution.

In order to form a glassy matrix or composition, the bioactive material and salt can be co-processed, such as in the absence of polyols. For example, spray-dried pharmaceuticals, such as, for example, insulin may be improved by the inclusion of low levels of a salt as defined herein, such as calcium lactate. The Tg of the composition and/or metal ion salt is preferably as defined herein. The metal ion salt may be amorphous and/or anhydrous and/or a lower hydrate or hydrate which has been treated so as to reduce the degree of hydration. The metal ion salt is present in the composition in an amount of less than or equal to 30, 20 or 10 wt.% based on the total weight of the composition, such as less than or equal to about 5 wt.%, 2 wt.% or 1 wt.%, for example from 1 to 10 wt.%, 2 to 8 wt.% or 3 to 6 wt.%.

The residual moisture content of the metal ion salt is preferably between 0 wt. % [totally anhydrous] to 50 wt. % [fully hydrated] based on the weight of the salt. The preferred moisture content, such as for calcium lactate, is from about 2 to 25 % preferably from about 10 to 20 % by wt. or 4 to 20 wt.% based on the weight of the salt.

In another embodiment of the invention, the composition is an amorphous powder, preferably a spray-dried amorphous powder. Typically, at least 50 percent by weight of the particles are amorphous in form, wherein crystalline forms make up less than 50 percent by weight of the total weight of the particles, regardless of the nature of individual particles. Preferably, at least 75 percent by weight of the particles are amorphous in form. More preferably, at least 90 percent by weight of the particles are amorphous in form, such as at least 95 wt.% or 99 wt.% based on the total weight of the powder. The compositions of the invention may be produced by, for example, spray-drying components separately or together followed by blending according to methods known in the art. Suitable conditions for forming glassy matrices comprising bioactive materials are known in the art.

In one embodiment, the composition is suitable for pulmonary administration. The compositions of the invention may, for example, be suitable for delivering bioactive materials topically to the lungs or systemically from the lungs. The compositions of the invention preferably comprise particles having a size distribution suitable for penetration into the deep lung area.

Tg refers to the glass transition temperature, as measured by differential scanning calorimetry (DSC) or DER. In the present invention, Tg is preferably taken to be the midpoint of the inflexion of the change of heat capacity (Cp) of the composition upon scanning through the transition when using DSC or DER. The glass transition temperature is the temperature range at which a composition changes from a glassy or vitreous state to a syrup or rubbery state. The Tg preferably refers to the initial Tg of the composition such as before exposure to an environment. Typically, the Tg values refer to a Tg measured at a residual moisture content or moisture content of, for example, less than 10wt.%, based on the total weight of the composition, such as less than 9, 8, 7, 6, 5, 4, 3, or 2 wt.%, or about from 1 to 5 wt.%, such as 2 to 4 wt.%.

In one embodiment, the glass transition temperature Tg of the composition is at least about 40°C, such as at least 50, 60, 70, 80, 90, 100, 110 or 120°C, such as from about 40 to 200°C, preferably from about 80 to 150°C or from about 110 to 130°C.

In another embodiment, the glass transition temperature Tg of the metal ion salt, either initially or after treatment, is at least about 60°C, such as from about 70 to 200°C, from about 110 to 190°C or from about 120 to 180°C. The molar mass of the metal ion salt is typically less than about 550 g/mol or 400 g/mol, such as less than 300 g/mol. It is unexpected that low molecular weight salts can provide such a high Tg.

In one embodiment of the invention, the change in Tg of the composition after 24 hours exposure at 25°C/60% RH is less than 25°C, such as less than 20°C, less than 15°C, or less than or equal to about 10°C, for example from 5 to 15°C.

The heat capacity, Cp J/(g.°C), provides a measure of the resistance of a composition to phase change, or in the case of a glassy composition, of devitrification. Cp can be measured using differential scanning calorimetry (DSC). The change in Cp from glassy to non-glassy state can be expressed as $\Delta C_p$. Typically, the composition of the invention, has a $\Delta C_p$ of at least about 1, preferably at least about 1.5, 2 or 2.5 J/(g.°C), such as from about 1 to 4 J/(g.°C) or from about 2 to about 3.5 J/(g.°C).

The metal ion salt, either initially or after treatment as described above, typically has a $\Delta C_p$ of at least about 1, preferably at least about 1.5, 2 or 2.5 J/(g.°C), such as from about 1 to 4 J/(g.°C) or from about 2 to about 3.5 J/(g.°C).

The composition of the invention is in the form of a powder. The powder may comprise particles in, for example, the

form of hollow spheres, discs or needles. The powder preferably comprises particles or microparticles as defined herein.

In one embodiment of the invention, the particles or microparticles are porous. The walls of the particles or microparticles of the invention may comprise pores, such as, for example, gaps, voids, spaces, or fissures.

In one embodiment, the particles or microparticles comprise one or more walls. The wall or walls of the particles or microparticles may be porous. For example the wall or walls of the particles or microparticles may be porous as described in WO 98/17257. The particles or microparticles according to the invention may have a wall thickness of no

more than 500 nm, such as from about 10 to 250 nm, or from about 100 to 150 nm.

In one embodiment, the walls of the particles or microparticles of the invention are non-porous i.e. are substantially free of pores, such as, for example, gaps, voids, spaces, fissures, for example, the pores comprise less than about 20 % or less than about 10% of the surface area of the microparticles. In this way, the particles or microparticles of the invention are not porous as described in WO 98/17257. In one embodiment of the invention, the walls of the particles or microparticles of the invention do not comprise an additional component which can subsequently be removed from the walls, for example, by treating the formed particles or microparticles with a solvent for the additional component. In another embodiment, the walls may comprise such an additional component.

In one embodiment, the particles, microparticles or powder of the invention, as described in any of the embodiments herein, provide a fine particle fraction (less than 6.5 μm or 5.8 μm) following aerosolization greater than about 20% or 25% or greater than about 35%, or greater than about 40%, 50%, 60% or 70% of the delivered dose. The Andersen Cascade Impactor or NGI may be used and the results analysed using Copley CITDAS software to determine the fine particle fraction and fine particle mass at different cut-off diameters e.g. <6.5 micron, <5.8 micron, <5 micron, < 3.3 micron and < 3 micron. The fine particle fraction is typically measured over a pressure drop of 4kPa.

In one embodiment of the invention, the fine particle fraction (less than 6.5 μm or 5.8 μm) is from about 20 to 90 %, from about 30 to 70 %, or from about 40 to 60%, such as from 70 or 80 to 90% of the delivered dose and/or the fine particle fraction (less than 5 μm) may be from about 30 to 60 %, or from about 40 to 50%, and/or the fine particle fraction (less than 3.3 or 3 μm) may be from about 10 to 90 %, from about 15 to 40, 50%, 60% or 70%, such as from about 20 to 30% or 50 to 80% of the delivered dose.

In one embodiment, the fine particle dose (less than 6.5 μm or 5.8 μm) is from about 20 to 90 %, from about 30 to 70 %, or from about 40 to 60%, such as from 70 or 80 to 90% of the initial dose and/or the fine particle fraction (less than 5 μm) may be from about 30 to 60 %, or from about 40 to 50%, and/or the fine particle fraction (less than 3.3 or 3 μm) may be from about 10 to 90 %, from about 15 to 40, 50% 60% or 70%, such as from about 20 to 30% or 50 to 80% of the initial dose in, for example, a blister or other receptacle.

In one embodiment of the invention, the particles, microparticles or powders are suitable for filling into a blister or other receptacle, or reservoir by machine or automated filling. The particles, microparticles or powders of the invention may be adapted for machine filling or automated filling. The particles, microparticles or powders may also have improved emptying performance from a blister, reservoir or other receptacle compared to particles, microparticles or powders not according to the invention.

The particles or microparticles of the invention may be hollow i.e. comprise one or more voids, filled with gas or air, with a surrounding wall-forming material. The wall-forming material may comprise the bioactive material and metal ion salt as described herein. In a preferred embodiment, the hollow microcapsules are not honeycombs as in maltesers. Alternatively, the hollow microcapsulres comprise honeycombs or multichambers, for example when the microcapsules are prepared from an emulsion.

The term "microparticles" means, in one embodiment, hollow particles enclosing a space, which space is filled with a gas or vapour but not with any solid materials. Honeycombed particles resembling the confectionery sold in the UK as "Maltesers" (Regd TM) are not formed. It is not necessary for the space to be totally enclosed (although this is preferred) and it is not necessary for the microparticles to be precisely spherical, although they are generally spherical. If the microparticles are not spherical, then the diameters referred to above relate to the diameter of a corresponding spherical microparticle having the same mass and enclosing the same volume of hollow space as the non-spherical microparticle.

The microparticles of the invention are preferably hollow particles comprising at least one wall enclosing one or more spaces, more preferably one wall enclosing one space.

In one embodiment, the microparticles of the invention are water-soluble i.e., have a solubility of at least about 0.1 $mg/cm^3$ in water at a temperature of 25°C, at least about 0.5 $mg/cm^3$, or at least about 1.0 $mg/cm^3$.

The composition comprises particles having a median geometric diameter of less than about 10μm. The particles according to the invention in any embodiment may have a median geometric diameter of less than about 5 μm. In one embodiment of the invention, the particles have a median geometric diameter of from about 1 to 2 μm, 1 to 5 μm or from 1.5 to 4.5, 1.75 to 4 or 2 to 3 or 3.5 μm, or from 3.5 to 9 μm or from about 4 to 8 μm such as from about 4 to 5, 6 or 7 μm. The median geometric diameter is measured at a dispersion pressure of 1.0 bar unless stated otherwise.

The median geometric diameter of the microparticles can be measured using a laser diffraction instrument (for example Helos KF, manufactured by Sympatec, Clausthal-Zellerfeld, Germany) as described in Example 1. Other instruments for measuring geometric particle diameter are well known in the art. The diameter of particles in a sample will range depending upon factors such as particle composition and methods of synthesis. The distribution of size of particles in a sample can be selected to permit optimal deposition to targeted sites within the respiratory tract.

The particles according to the invention in any embodiment typically have a mass median aerodynamic diameter (MMAD) of equal to or less than about 10 μm, such as from about 0.1 to 10 μm.

In one embodiment, the MMAD is from about 1 μm to about 5 or 6 μm. In another embodiment of the invention,

the MMAD is from about 1 μm to about 3 μm. In a further embodiment, the MMAD is from about 2, 3 or 4 μm to about 5 or 6 μm such as from 2 to 4 or 2 to 3 μm.

Experimentally, aerodynamic diameter can be determined by employing a gravitational settling method, whereby the time for an ensemble of particles to settle a certain distance is used to infer directly the aerodynamic diameter of the particles. An indirect method for measuring the mass median aerodynamic diameter (MMAD) is the Andersen Cascade Impactor (ACI). The particle size may also be determined using a Next Generation Impactor (NGI).

In another embodiment, the composition is in the form of a dry powder which is preferably free-flowing. For example, the composition is typically free-flowing at 25°C/60% RH for an extended duration, such as at least 24 hours and/or 40°C/75%RH for a duration of at least 30 minutes, or at least 1, 2, 4, or more hours, preferably more than 6 hours.

The Carr's Index is based on the decrease in powder volume during tapping and can be used to predict flowability (R.L. Carr, (1965), Chem Eng. 72, 163-168). The lower the number, the more free-flowing the powder. An increase in the value is proportional to adhesion and friction properties of a powder, including (attractive) triboelectric charge.

Carr's Index (or Carr's Compressibility Index), C, can be calculated using the following formulae:

$$C = 100 \text{ x } (V_o\text{-}V_f)/V_o \text{ or } 100 \text{ x } (D_r D_o)/D_o$$

where "V" and "D" represent powder volume and density respectively, subscript "o" denotes the initial or untapped state and "f" the final or tapped state.

The particles or powder according to the invention preferably have a Carr's Index of less than about 30%, such as less than about 26%, 25% or 23%, or from about 5% to 30%, from about 10% to 23% or 26%, or from about 15 or 19 to 23 or 26% or from about 20 to 26% and optionally the particles have a median geometric diameter of, from about 2 or 3.5 to 9 μm, or from about 3 or 4 to 8 μm, such as from about 1 to 5 μm, 1.5 to 4.5, 1.75 to 4 or 2 to 3 or 3.5 μm, 2 to 2.7 μm or from 4 to 5, 6, 7 or 8 μm.

The matrix may comprise a solid solution of the at least one bioactive material, where the bioactive material is effectively dissolved in the matrix material, or the bioactive material may be present in suspension in the matrix. For example, the bioactive material may be present as a solid dispersion in the glass.

Typically, the bioactive material is present in a solid, not liquid, form in the composition or matrix and is delivered as a solid form. For example, the composition of the invention is not delivered as a solution or liquid, such as prepared by reconstitution or dissolving the composition.

The composition is in the form of a powder. For inhalation, the composition is generally in the form of a powder. The powder may be a dry powder. By "dry" it is intended to mean that the moisture content of the powder is less than about 10 wt.%, such as less than about 5, 4, 3, 2 or 1 wt.%. Typical moisture contents are from about 0.5 to 4 wt.% or 1 to 3 wt.% based on the weight of the powder.

In one embodiment, the composition is a pharmaceutical composition which may or may not comprise one or more pharmaceutically acceptable excipients. Typically the composition does not comprise excipients.

Examples of bioactive materials include pharmaceutically effective substances, including anti-inflammatory drugs, analgesics, antiarthritic drugs, anti-cancer, anticoagulants, antispasmodics, antidepressants, antipsychotics, tranquilizers, antianxiety drugs, narcotic antagonists, antiparkinsonism agents, cholinergic agonists, chemotherapeutic drugs, immunosuppressive agents, antiviral agents, antimicrobial agents, antifungal agents, appetite suppressants, anticholinergics, antiemetics, antihistaminics, antimigraine agents, coronary, cerebral or peripheral vasodilators, hormonal agents, contraceptives, antithrombotic agents, diuretics, antihypertensive agents, cardiovascular drugs, opioids, anti-obesity agents; diagnostics and gene therapies.

In one embodiment of the invention, the composition may comprise one or more therapeutic agents selected from 13-cis-retinoic acid, 5-fluorouracil, 2-pentenylpenicillin, L-alphaacetylmethadol, ablukast, S-adenosylmethionine, acebutolol, aceclofenac, acetaminophen, acetaphenazine, acetophenazine, aclidinium, acrivastine, acyclovir, ademetionine, adenosine receptor agonists or antagonists, e.g. adenosine 2α agonists, adinazolam, adrafinil, ahnotriptan, albuterol, albuterol, albuterol sulfate, alfentanil, alfentanil HCI, alizapride, allylprodine, alminoprofen, almotriptan, alperopride, alpha-interferon, alphaprodine, alpidem, alprazolam, alseroxlon, amantadine, ambrisentan, amesergide, amfenac, aminoglycosides, aminopropylon, amiodarone HCl, amisulpride, amitriptyline, amixetrine, amlodipine, amoxapine, amoxicillin, amperozide, amphenidone, amphetamine, amphotericin B, ampicillin, amylpenicillin, andolast, andropinirole, anileridine, anthracyclines, antibacterial agents and agents for cystic fibrosis and/or tuberculosis treatment, e.g. Pseudomonas aeruginosa infection vaccines (eg Aerugen®), mannitol, denufosol, glutathione, N-acetylcysteine, amikacin duramycin, gentamycin, tobramycin, dornase alfa, alpha 1 -antitrypsin, heparin, dextran, capreomycin, vancomycin, meropenem, ciprofloxacin, piperacillin; apazone, apomorphine, apomorphine hydrochloride, apomorphine diacetate, astemizole, atenolol, atropine sulfate, azacyclonol, azasetron, azatadine, azathioprine, azelastine, azidocillin, aztreonam, Bacille Calmette-Guerin, baclofen, bambuterol, beclomethasone dipropionate, bemiparin, benactyzine, benmoxine, benoxaprofen, benperidol, benserazide, benzpiperylon, benzquinamide, benztropine, benzydramine, benzylmorphine,

benzylpenicillin, beta-2 integrin antagonists, beta-lactams, beta-interferon, bezitramide, binedaline, biperiden, bitolterol, bitolterol mesylate, blood factors and blood factor constructs, eg FVIII-Fc and FIX-Fc; brofaromine, bromfenac, bromisovalum, bromocriptine, bromopride, bromperidol, brompheniramine, broxaterol, brucine, buclizine, budesonide, formoterol fumarate, budipine, bufexamac, buprenorphine, bupropion, buramate, buspirone, butaclamol, butaperazine, butixocort butorphanol, butriptyline, cabergoline, caffeine, calcium-N-carboamoylaspartate, calcium, channel blockers, eg gallopamil, cannabinoids, captodiamine, capuride, carbamazepine, carbcloral, carbenicillin, carbidopa, carbiphene, carbromal, carfecillin, carindacillin, carmoterol, caroxazone, carphenazine, carpipramine, carprofen, cefazolin, cefinetazole, cefmetazole, cefoxitin, ceftazidime, cephacetrile, cephalexin, cephaloglycin, cephaloridine, cephalosporin C, cephalosporins, cephalotin, cephamycin A, cephamycin B, cephamycin C, cephamycins, cepharin, cephradine, cericlamine, cetrizine, cetirizene, chloralbetaine, chlordiazepoxide, chlorobutinpenicillin, chlorpheniramine, chlorpromazine, chlorprothixene, choline, cialis, ciclesonide, cidofovir, cilazaprol, cilostazol, cinchophen, cinmetacin, cinnarizine, cipramadol, ciprofloxacin, cisplatin, citalopram, clebopride, clemastine, clenbuterol, clobazam, clobenzepam, clocapramine, clomacran, clometacin, clometocillin, clomipramine, clonazepam, clonidine, clonitazene, clonixin, clopenthixol, clopriac, clospirazine, clothiapine, clovoxamine, cloxacillin, clozapine, codeine, colistimethate, cotinine, cromoglycate, cromolyn sodium, cyamemazine, cyclacillin, cyclizine, cyclobenzaprine, cyclosporin A, cyproheptadine, cytokine antagonists, eg chemokine antagonists and inhibitors and modifiers of cytokine synthesis including modifiers and inhibitors of the proinflammatory transcription factor, NFkB; deprenyl, desflurane, desipramine, dexamethasone sodium phosphate, desloratidine, dexfenfluramine, dexmedetomidine, dexomethasone, dextroamphetamine, dextromoramide, dextropropoxyphene, diamorphine, diazepam, diclofenac, dicloxacillin, dihydrocodeine, dihydroergokryptine, dihydroergotamine, diltiazem, diphenhydramine, diphenicillin, diphenidol, diphenoxylate, dipipanone, disulfiram, docetaxel, dolasetronmethanesulfonate, domeridone, doripenem, dornase alfa, dosulepin, doxepin, doxorubicin, doxylamine, dronabinol, droperidol, droprenilamin HCl, duloxetine, eletriptan, eliprodil, enalapril, enciprazine, endothelin-receptor antagonists, e.g. LU-135252, enflurane, enoxaparin, entacapone, entonox, enzymes, ephedrine, epinephrine, epirubicin, eptastigmine, ergolinepramipexole, ergotamine, ergotamine tartrate, etamiphyllin, etaqualone, ethambutol, ethoheptazine, ethylnorepinephrine, etodolac, exendins, factor VII, factor VIII, factor IX, factor XIII, famciclovir, famotidine, fenfluramine, fenleuton, fenoterol, fentanyl, fexofenadine, fibrinogen, fientanyl, flesinoxan, fluconazole, flunisolide, fluoroquinolones, fluoxetine, flupenthixol, fluphenazine, flurazepam, flupirtine, flurazepam, fluspirilene, fluticasone propionate, fluvoxamine, formoterol fumarate, foscarnet, frovatriptan, gabapentin, galanthamine, gamma-interferon, ganciclovir, gepirone, ghrelin, glucagons, GLP-1, glutathione, glycopyrrolate, glycopyrronium, granisetron, haloperidol, halothane, heliox, heparin, heparin sodium, heparin sulphate, heptylpenicillin, hetacillin, hGH, hydromorphone, hydroxychlorquine, hydroxyzine, hyoscine, ibuprofen, idarubicin, idazoxan, IGF-1, iloprost, imipramine, indacaterol, indoprofen, inducible nitric oxide synthase (iNOS) inhibitors; insulin (recombinant human), insulin aspart, insulin glulisine; insulin lispro, neutral, regular and soluble insulins, isophane insulins, insulin zinc, protamine zinc insulin, insulin analogues, acylated insulin, insulin glargine, insulin detemir; interleukins and inhibitors of interleukins, eg aldesleukin; ipratropium bromide, iproniazid, ipsapiraone, iralukast, isocarboxazid, isoetharine hydrochloride, isoflurane, isometheptene, isoniazid, rifampin, parathyroid hormone and analogues (eg Ostabolin-C); pyrazinamide, ethambutol, icodextrin, indacaterol, indinavir, isoprenaline isoproterenol, isoproterenol hydrochloride, isoproterenol bitartrate, isosorbide dinitrate, itraconazole, ketamine, ketoprofen, ketorolac, ketotifen, kitanserin, lazabemide, leptin, lesopitron, levalbuterol hydrochloride, levocabastine, levodopa, leflunomide, leuprolide, levofloxacin, levorphanol, lidocaine, lisinopril, lisuride, lofentanil, lofepramine, lomustine, loprazolam, loratidine, lorazepam, lorezepam, loxapine, macrolides, maprotoline, mazindol, mazipredone, meclofenamate, mecloqualone, medetomidine, medifoxamine, melperone, memantine, menthol, meperidine, meperidine HCl, meptazinol, meropenem, mesoridazine, metampicillin, metaproterenol, metaproterenol sulfate, methacholine chloride, methadone, methaqualone, methicillin, methotrexate, methprylon, methsuximide, methyphenidate, methyprylon, methysergide, metoclopramide, metofenazate, metomidate, metopimazine, metopon, metoprolol, metralindole, mianserin, midazolam, milnacipran, minaprine, mirtazapine, mizolastine, moclobemide; mofegiline, molindrone, mometasone, mometasone furoate, monteleukast, morphine, muscarinic receptor (M1, M2, and M3) antagonists; mucolytic agents for the treatment of COPD and cystic fibrosis, eg N-acetylcysteine, and ambroxol; mycophenolate mofetil, nacestelyn, nabilone, nadolol, nafcillin, nalbuphine, nalmefene, nalorphine, naloxone, naltrexone, naratriptan, naphazoline, natamycin, nedocromil sodium, nefazodone, nefopam, nelfinavir, nicergoline, nicotine, nicotine, nifedipine, nisoxetine, nitrous oxide, nitroglycerin, nomifensine, nortriptyline, nucleoside reverse transcriptase inhibitors (eg didanosine, lamivudine, stavudine, zalcitabine, and zidovudine) and non-nucleoside reverse transcriptase inhibitors (eg nevirapine and efavirenz); nucleic acids, NVA237, nystatin, obestatin, olanzapine, omoconazole, ondansetron, ontazolast, orciprenaline, orphenadrine, oseltamivir, osteoporosis agents, eg bisphosphonates; oxitropium, oxprenolol, oxybutynin, oxycodone, oxymetazoline, paclitaxel, palonosetron, papaveretum, papaverine, paroxetine, pemoline, penfluridol, penicillins, penicillin N, penicillin O, penicillin S, penicillin V, pentamidine isethionate, pentazocine, pentetate, calcium trisodium, pentetate, zinc trisodium, pentobarbital, peptides, peramivir, pergolike, pericyazine, perphenazine, pethidine, phenazocine, phenelzine, phenobarbital, phentermine, phentolamine, phenyhydrazine, phenylephrine, phenylpropanolamine, phosphodiesterase-5, phosphodiesterase (PDE) inhibitors, eg methylxanthines, theophylline, aminophylline, choline theophyllinate, and selective PDE

isoenzyme inhibitors, PDE 3 inhibitors, eg milrinone and motapizone; PDE 4 inhibitors, eg rolipram, cilomilast, roflumilast, oglemilast, and ONO 6126; PDE 3/4 inhibitors, eg zardaverine and tolafentrine; inducers of HDAC2 eg theophylline; picumeterol, pilocarpine, pimozide, pipamerone, piperacetazine, pipotiazine, pirbuterol acetate, pirenzipine, pirbuterolnaloxone, piroxicam, pirprofen, pitavastatin, pizotifen, pizotyline, pleconaril, polypeptides, polypeptide YY, posaconazole, pramipexole, pranlukast, prentoxapylline, procaine, procaterol HCl, prochlorperazine, procyclidine, promazine, promethazine, propacetamol, propanolol, propentofylline, propofol, propoxyphene, propranolol, propylhexidine, proteins, protriptyline, pseudoephedrine, quetiapine, quinine, rasagiline, reboxetine, remacemide, remifentanil, remoxipride, reproterol, retinol, ribavirin, rimiterol, rimantadine, rimonabant, risperidone, ritanserin, ritodrine, ritnoavir, rizatriptan, rofleponide, roxindole, ruprintrivir, salicylate, salbutamol, salmeterol xinafoate, salmeterol, saquinavir, scopolamine, selegiline, sertindole, sertraline, sevoflurane, sibutramine, sildenafil, sirolimus, spheramine, spiperone, streptomycin, sulphonamides, sufentanil, sulpiride, sumatriptan, tacrolimus, tadalafil, tandospirone, taxanes, telenzipine, terbutaline, terguride, testosterone, testosterone acetate, estosterone enanthate, terfenadine, tepoxalin, terileflunomide, testosterone proprionate, tetracyclines, tetrahydrocannabinol, tetrahydrozoline, thioridazine, thiothixene, thrombin, tiagabine, tianeptine, timolol, tiotropium bromide monohydrate, tiotropium, tizanidine, tobramycin, tofenacin, tolcapone, tolfenamate, tolfenamicacid, topiramate, tramadol, tramazoline, tranylcypromine, trazadone, trehalose, triamcinolone acetonide, triethylperazine, trifluoperazine, trifluperidol, triflupromazine, trihexyphenidyl, trimeprazine, trimethobenzamide, trimipramine, tropisetron, tryptase and elastase inhibitors, tryptophan, vaccine antigens, valacyclovir, valproicacid, vardenafil, venlafaxine, verapamil, verlukast, vigabatrin, viloxazine, vinca alkaloids, vincristine, voriconazole, VR776, vWF, xylometazoline, yohimbine, zafirlukast, zalospirone, zanamivir, zileuton, ziprasidone, zolazepam, zolmitriptan, zolpidem, zopiclone, zotepine, zuclopenthixol, and salts and combinations thereof.

It will be clear to a person skilled in the art that, where appropriate, the medicaments may be linked to a carrier molecule or molecules and/or used in the form of prodrugs, salts, as esters, or as solvates to optimise the activity and/or stability of the medicament.

Compositions according to the invention may also be used to deliver combinations of two or more different medicaments. Specific combinations of two medicaments which may be mentioned include combinations of steroids and $\beta_2$-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; glycopyrrolate and indacaterol; mometasone and indacaterol; and mometasone and formoterol. Specifically, compositions according to the invention may also be used to deliver combinations of three different medicaments.

It is also envisaged that the pharmaceutical composition may comprise one or more, preferably one, anticholinergic 1, optionally in combination with a pharmaceutically acceptable excipient.

The anticholinergic **1** can be selected from the group consisting of

a) tiotropium salts **1a,**

b) compounds of formula **1c**

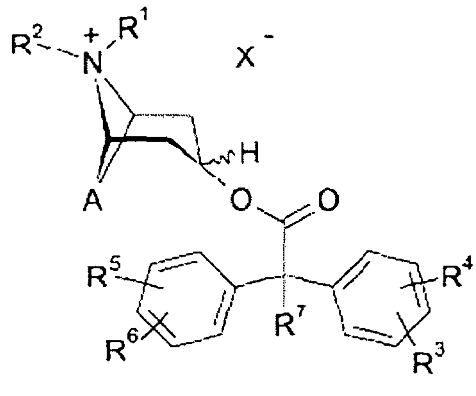

**1c**

wherein

A denotes a double-bonded group selected from among

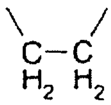 , 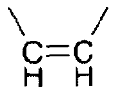 and 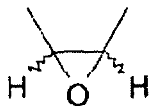 ;

$X^-$ denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,

$R^1$ and $R^2$ which may be identical or different denote a group selected from among methyl, ethyl, n-propyl and iso-propyl, which may optionally be substituted by hydroxy or fluorine, preferably unsubstituted methyl;

$R^3$, $R^4$, $R^5$ and $R^6$, which may be identical or different, denote hydrogen, methyl, ethyl, methyloxy, ethyloxy, hydroxy, fluorine, chlorine, bromine, CN, $CF_3$ or $NO_2$;

$R^7$ denotes hydrogen, methyl, ethyl, methyloxy, ethyloxy, $-CH_2-F$, $-CH_2-CH_2-F$, $-0-CH_2-F$, $-0-CH_2-CH_2-F$, $-CH_2-OH$, $-CH_2-CH_2-OH$, $CF_3$, $-CH_2-OMe$,$-CH_2-CH_2-OMe$, $-CH_2-OEt$, $-CH_2-CH_2-OEt$, -O-COMe, -O-COEt, $-Q-COCF_3$, $-Q-COCF_3$, fluorine, chlorine or bromine;

c) compounds of formula **1d**

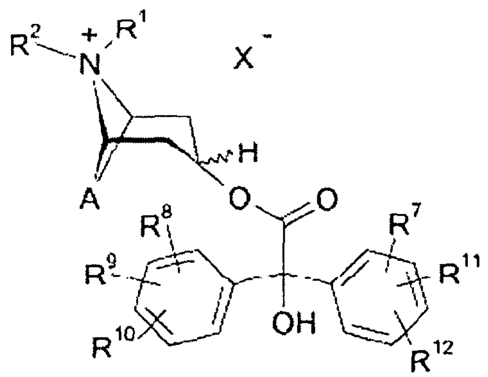

**1d**

wherein
A, $X^-$, $R^1$ and $R^2$ may have the meanings as mentioned hereinbefore and wherein $R^7$, $R^8$, $R^9$, $R^{10}$, $R^{11}$ and $R^{12}$, which may be identical or different, denote hydrogen, methyl, ethyl, methyloxy, ethyloxy, hydroxy, fluorine, chlorine, bromine, CN, $CF_3$ or $NO_2$, with the proviso that at least one of the groups $R^7$, $R^8$, $R^9$, $R^{10}$, $R^{11}$ and $R^{12}$ is not hydrogen,

d) compounds of formula **1e**

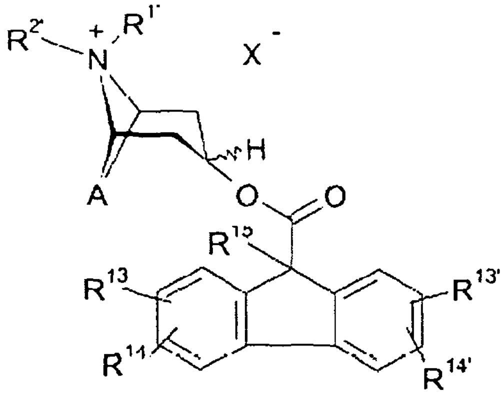

**1c**

wherein A and $X^-$ may have the meanings as mentioned hereinbefore, and wherein $R^{15}$ denotes hydrogen,

hydroxy, methyl, ethyl, $-CF_3$, $CHF_2$ or fluorine;
$R^{1'}$ and $R^{2'}$ which may be identical or different denote $C_1$-$C_5$-alkyl which may optionally be substituted by $C_3$-$C_6$-cycloalkyl, hydroxy or halogen, or
$R^{1'}$ and R2' together denote a $-C_3$-$C_5$-alkylene-bridge;
$R^{13}$, $R^{14}$, $R^{13'}$ and $R^{14'}$ which may be identical or different denote hydrogen, $-C_1$-$C_4$-alkyl, $-C_1$-$C_4$-alkyloxy, hydroxy, $-CF_3$, $-CHF_2$, CN, $NO_2$ or halogen,

e) compounds of formula **1f**

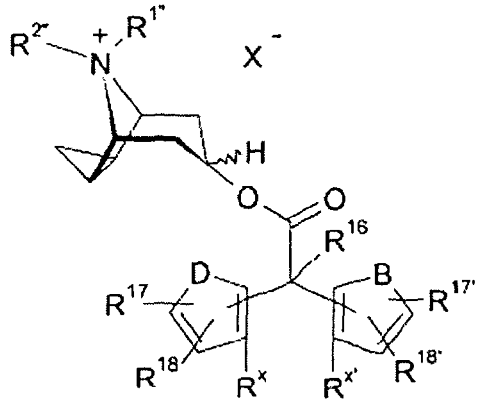

**1f**

wherein $X^-$ may have the meanings as mentioned hereinbefore, and wherein

D and B which may be identical or different, preferably identical, denote -O, -S, -NH,$-CH_2$, -CH=CH, or $-N(C_1$-$C_4$-alkyl)-;

$R^{16}$ denotes hydrogen, hydroxy, $-C_1$-$C_4$-alkyl, $-C_1$-$C_4$-alkyloxy, $-C_1$-$C_4$ -alkylene-Halogen, $-O-C_1$-$C_4$ alkylene-halogen, $-C_1$-$C_4$-alkylene-OH, $-CF_3$, $CHF_2$, $-C_1$-$C_4$-alkylene-$C_1$-$C_4$ alkyloxy, -O- $COC_1$ $-C_4$-alkyl, $-O-COC_1$ $-C_4$-alkylene-halogen, $-C_1$-$C_4$-alkylene-$C_3$-$C_6$-cycloalkyl, $-O-COCF_3$ or halogen;

$R^{1'}$ and $R^{2'}$ which may be identical or different, denote $-C_1$-$C_5$-alkyl, which may optionally be substituted by $-C_3$-$C_6$-cycloalkyl, hydroxy or halogen, or

$R^{1'}$ and $R^{2'}$ together denote a -C3-C5-alkylene bridge;

$R^{17}$, $R^{18}$, $R^{17}$ and $R^{18'}$, which may be identical or different, denote hydrogen, $C_1$-$C_4$-alkyl, $C_1$-$C_4$-alkyloxy, hydroxy, $-CF_3$, $-CHF_2$, CN, $NO_2$ or halogen;

$R^x$ and $R^{x'}$ which may be identical or different, denote hydrogen, $C_1$-$C_4$-alkyl, $C_1$-$C_4$-alkyloxy, hydroxy, $-CF_3$, $-CHF_2$, CN, $NO_2$ or halogen or

$R^x$ and $R^{x'}$ together denote a single bond or a bridging group selected from among the bridges -O, -S, -NH, $-CH_2$, $-CH_2$-$CH_2$-, $-N(C_1$-$C_4$-alkyl), $-CH(C_1$-$C_4$-alkyl)- and-$C(C_1$-$C_4$-alkyl$)_2$, and

f) compounds of formula **1g**

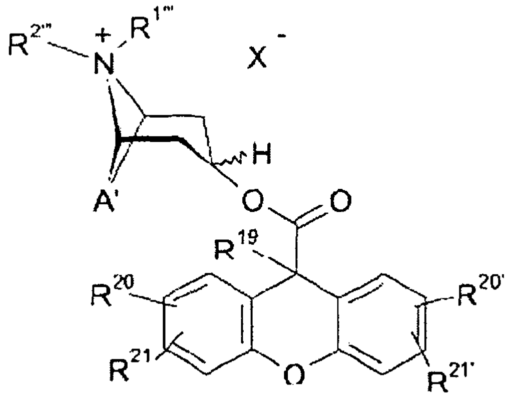

1g

wherein $X^-$ may have the meanings as mentioned hereinbefore, and wherein A' denotes a double-bonded group selected from among

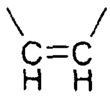 and 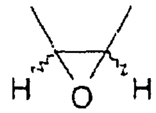 ;

$R^{19}$ denotes hydroxy, methyl, hydroxymethyl, ethyl, $-CF_3$, $CHF_2$ or fluorine;
$R^{1'''}$ and $R^{2'''}$ which may be identical or different denote $C_1$-$C_5$-alkyl which may optionally be substituted by $C_3$-$C_6$-cycloalkyl, hydroxy or halogen, or
$R^{1'''}$ and $R^{2'''}$ together denote a $-C_3$-$C_5$-alkylene-bridge;
$R^{20}$, $R^{21}$, $R^{20'}$ and $R^{21'}$ which may be identical or different denote hydrogen, $-C_1$-$C_4$-alkyl, $-C_1$-$C_4$-alkyloxy, hydroxy, $-CF_3$, $-CHF_2$, CN, $NO_2$ or halogen.

The compounds of formula 1c are known in the art (WO 02/32899).

In a preferred embodiment of the invention the method comprises administration of compounds of formula **1c,** wherein
$X^-$ denotes bromide;
$R^{1'}$ and $R^2$ which may be identical or different denote a group selected from methyl and ethyl, preferably methyl;
$R^3$, $R^4$, $R^5$ and $R^6$, which may be identical or different, denote hydrogen, methyl, methyloxy, chlorine or fluorine;
$R^7$ denotes hydrogen, methyl or fluorine, optionally together with a pharmaceutically acceptable excipient.

Of particular importance are compounds of general formula **1c,** wherein A denotes a double-bonded group selected from among

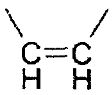 and 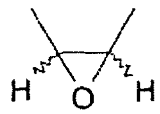

The compounds of formula **1c,** may optionally be administered in the form of the individual optical isomers, mixtures of the individual enantiomers or racemates thereof.

Of particular importance within a method according to the invention are the following compounds of formula **1c:**

tropenol 2,2-diphenylpropionic acid ester methobromide,
scopine 2,2-diphenylpropionic acid ester methobromide,
scopine 2-fluoro-2,2-diphenylacetic acid ester methobromide and
tropenol 2-fluoro-2,2-diphenylacetic acid ester methobromide.

The compounds of formula **1d** are known in the art (WO 02/32898).

In a preferred embodiment of the invention the method comprises administration of compounds of formula **1d,** wherein
A denotes a double-bonded group selected from among

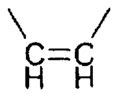 and 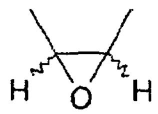 ;

$X^-$ denotes bromide;
$R^{1'}$ and $R^2$ which may be identical or different denote methyl or ethyl, preferably methyl;
$R^7$, $R^8$, $R^9$, $R^{10}$, $R^{11}$ and $R^{12}$, which may be identical or different, denote hydrogen, fluorine, chlorine or bromine, preferably fluorine with the proviso that at least one of the groups $R^7$, $R^8$, $R^9$, $R^{10}$, $R^{11}$ and $R^{12}$ not hydrogen, optionally together with a pharmaceutically acceptable excipient.

Of particular importance within the method according to the invention are the following compounds of formula **1d:**

tropenol 3,3',4,4'-tetrafluorobenzilic acid ester methobromide,
scopine 3,3',4,4'-tetrafluorobenzilic acid ester methobromide,
scopine 4,4'-difluorobenzilic acid ester methobromide,
tropenol 4,4'-difluorobenzilic acid ester methobromide,
scopine 3,3'-difluorobenzilic acid ester methobromide, and
tropenol 3,3'-difluorobenzilic acid ester methobromide.

The pharmaceutical compositions according to the invention may contain the compounds of formula **1d** optionally in the form of the individual optical isomers, mixtures of the individual enantiomers or racemates thereof.

The compounds of formula **1e** are known in the art (WO 03/064419).

In a preferred embodiment of the invention the method comprises administration of compounds of formula **1e,** wherein
A denotes a double-bonded group selected from among

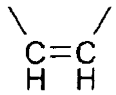 and 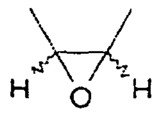 ;

$X^-$ denotes an anion selected from among chloride, bromide and methanesulphonate, preferably bromide;
$R^{15}$ denotes hydroxy, methyl or fluorine, preferably methyl or hydroxy;
$R^{1'}$ and $R^{2'}$ which may be identical or different represent methyl or ethyl, preferably methyl;
$R^{13}$, $R^{14}$, $R^{13'}$ and $R^{14'}$ which may be identical or different represent hydrogen, $-CF_3$, $-CHF_2$ or fluorine, preferably hydrogen or fluorine, optionally together with a pharmaceutically acceptable excipient.

In another preferred embodiment of the invention the method comprises administration of compounds of formula **1e,** wherein
A denotes a double-bonded group selected from among

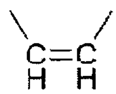 and 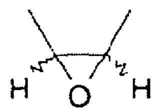 ;

$X^-$ denotes bromide;
$R^{15}$ denotes hydroxy or methyl, preferably methyl;
$R^{1'}$ and $R^{2'}$ which may be identical or different represent methyl or ethyl, preferably methyl;
$R^{13}$, $R^{14}$, $R^{13'}$ and $R^{14'}$ which may be identical or different represent hydrogen or fluorine, optionally together with a pharmaceutically acceptable excipient.

Of particular importance within the method according to the invention are the following compounds of formula **1e:**

tropenol 9-hydroxy-fluorene-9-carboxylate methobromide ;
tropenol 9-fluoro-fluorene-9-carboxylate methobromide;
scopine 9-hydroxy-fluorene-9-carboxylate methobromide;
scopine 9-fluoro-fluorene-9-carboxylate methobromide;
tropenol 9-methyl-fluorene-9-carboxylate methobromide;
scopine 9-methyl-fluorene-9-carboxylate methobromide.

The pharmaceutical compositions according to the invention may contain the compounds of formula **1e** optionally in the form of the individual optical isomers, mixtures of the individual enantiomers or racemates thereof.

The compounds of formula **1f** are known in the art (WO 03/064418).

In another preferred embodiment of the invention the method comprises administration of compounds of formula **1f** wherein

$X^-$ denotes chloride, bromide, or methanesulphonate, preferably bromide;
D and B which may be identical or different, preferably identical, denote -O, -S, -NH or - CH=CH-;
$R^{16}$ denotes hydrogen, hydroxy, -$C_1$-$C_4$-alkyl, -$C_1$-$C_4$ alkyloxy, -$CF_3$, -$CHF_2$, fluorine, chlorine or bromine;
$R^{1''}$ and $R^{2''}$ which may be identical or different, denote $C_1$-$C_4$-alky, which may optionally be substituted by hydroxy, fluorine, chlorine or bromine, or
$R^{1''}$ and $R^{2''}$ together denote a -$C_3$-$C_4$-alkylene-bridge;
$R^{17}$, $R^{18}$, $R^{17'}$ and $R^{18'}$, which may be identical or different, denote hydrogen, $C_1$-$C_4$ -alkyl, $C_1$-$C_4$-alkyloxy, hydroxy, -$CF_3$, -$CHF_2$, CN, $NO_2$, fluorine, chlorine or bromine;
$R^x$ and $R^{x'}$ which may be identical or different, denote hydrogen, $C_1$-$C_4$-alkyl, $C_1$-$C_4$-alkyloxy, hydroxy, -$CF_3$, -$CHF_2$, CN, $NO_2$, fluorine, chlorine or bromine or
$R^x$ and $R^{x'}$ together denote a single bond or a bridging group selected from among the bridges -O, -S, -NH- and -$CH_2$-, optionally together with a pharmaceutically acceptable excipient.

In another preferred embodiment of the invention the method comprises administration of compounds of formula **1f,** wherein

$X^-$ denotes chloride, bromide, or methanesulphonate, preferably bromide;
D and B which may be identical or different, preferably identical, denote -S or-CH=CH-;
$R^{16}$ denotes hydrogen, hydroxy or methyl;
$R^{1''}$ and $R^{2''}$ which may be identical or different, denote methyl or ethyl;
$R^{17}$, $R^{18}$, $R^{17}$ and $R^{18'}$, which may be identical or different, denote hydrogen, -$CF_3$ or fluorine, preferably hydrogen;
$R^x$ and $R^{x'}$ which may be identical or different, denote hydrogen, -$CF_3$ or fluorine, preferably hydrogen or
$R^x$ and $R^{x'}$ together denote a single bond or the bridging group -O-, optionally together with a pharmaceutically acceptable excipient.

In another preferred embodiment of the invention the method comprises administration of compounds of formula **If** wherein

$X^-$ denotes bromide;
D and B denote -CH=CH-;
$R^{16}$ denotes hydrogen, hydroxy or methyl;
$R^{1''}$ and $R^{2''}$ denote methyl;
$R^{17}$, $R^{18}$, $R^{17'}$ and $R^{18'}$, which may be identical or different, denote hydrogen or fluorine, preferably hydrogen;
$R^x$ and $R^{x'}$ which may be identical or different, denote hydrogen or fluorine, preferably hydrogen or
$R^x$ and $R^{x'}$ together denote a single bond or the bridging group -O-, optionally together with a pharmaceutically acceptable excipient.

Of particular importance within the method according to the invention are the following compounds of formula **1f:**

cyclopropyltropine benzilate methobromide;
cyclopropyltropine 2,2-diphenylpropionate methobromide;
cyclopropyltropine 9-hydroxy-xanthene-9-carboxylate methobromide;
cyclopropyltropine 9-methyl-fluorene-9-carboxylate methobromide;
cyclopropyltropine 9-methyl-xanthene-9-carboxylate methobromide;
cyclopropyltropine 9-hydroxy-fluorene-9-carboxylate methobromide;
cyclopropyltropine methyl 4,4'-difluorobenzilate methobromide.

The pharmaceutical compositions according to the invention may contain the compounds of formula **1f** optionally in the form of the individual optical isomers, mixtures of the individual enantiomers or racemates thereof.

The compounds of formula **1g** are known in the art (WO 03/064417).

In another preferred embodiment of the invention the method comprises administration of compounds of formula **1g** wherein

A' denotes a double-bonded group selected from among

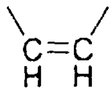 and 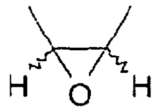 ;

X- denotes chloride, bromide or methanesulphonate, preferably bromide;
$R^{19}$ denotes hydroxy or methyl;
$R^{1'''}$ and $R^{2'''}$ which may be identical or different represent methyl or ethyl, preferably methyl;

$R^{20}$, $R^{21}$, $R^{20'}$ and $R^{21'}$ which may be identical or different represent hydrogen, $-CF_3$,$-CHF_2$ or fluorine, preferably hydrogen or fluorine, optionally together with a pharmaceutically acceptable excipient.

In another preferred embodiment of the invention the method comprises administration of compounds of formula **1g** wherein
A' denotes a double-bonded group selected from among

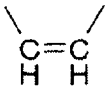 and 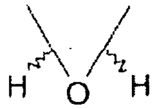 ;

$X^-$ denotes bromide;
$R^{19}$ denotes hydroxy or methyl, preferably methyl;
$R^{1'''}$ and $R^{2'''}$ which may be identical or different represent methyl or ethyl, preferably methyl;
$R^3$, $R^4$, $R^{3'}$ and $R^{4'}$ which may be identical or different represent hydrogen or fluorine, optionally together with a pharmaceutically acceptable excipient.
Of particular importance within the method according to the invention are the following compounds of formula **1g:**

tropenol 9-hydroxy-xanthene-9-carboxylate methobromide;
scopine 9-hydroxy-xanthene-9-carboxylate methobromide;
tropenol 9-methyl-xanthene-9-carboxylate methobromide;
scopine 9-methyl-xanthene-9-carboxylate methobromide;
tropenol 9-ethyl-xanthene-9-carboxylate methobromide;
tropenol 9-difluoromethyl-xanthene-9-carboxylate methobromide;
scopine 9-hydroxymethyl-xanthene-9-carboxylate methobromide.

The pharmaceutical compositions according to the invention may contain the compounds of formula **1g** optionally in the form of the individual optical isomers, mixtures of the individual enantiomers or racemates thereof.
The alkyl groups used, unless otherwise stated, are branched and unbranched alkyl groups having 1 to 5 carbon atoms. Examples include: methyl, ethyl, propyl or butyl. The groups methyl, ethyl, propyl or butyl may optionally also be referred to by the abbreviations Me, Et, Prop or Bu. Unless otherwise stated, the definitions propyl and butyl also include all possible isomeric forms of the groups in question. Thus, for example, propyl includes n- propyl and iso-propyl, butyl includes iso-butyl, sec. butyl and tert. -butyl.
The cycloalkyl groups used, unless otherwise stated, are alicyclic groups with 3 to 6 carbon atoms. These are the cyclopropyl, cyclobutyl, cyclopentyl and cyclohexyl groups. According to the invention cyclopropyl is of particular importance within the scope of the present invention.
The alkylene groups used, unless otherwise stated, are branched and unbranched double- bonded alkyl bridges with 1 to 5 carbon atoms. Examples include: methylene, ethylene, propylene or butylene.
The alkylene-halogen groups used, unless otherwise stated, are branched and unbranched double-bonded alkyl bridges with 1 to 4 carbon atoms which may be mono-, di- or trisubstituted, preferably disubstituted, by a halogen. Accordingly, unless otherwise stated, the term alkylene-OH groups denotes branched and unbranched double-bonded alkyl bridges with 1 to 4 carbon atoms which may be mono-, di- or trisubstituted, preferably monosubstituted, by a hydroxy.
The alkyloxy groups used, unless otherwise stated, are branched and unbranched alkyl groups with 1 to 5 carbon atoms which are linked via an oxygen atom. The following may be mentioned, for example: methyloxy, ethyloxy, propyloxy or butyloxy. The groups methyloxy, ethyloxy, propyloxy or butyloxy may optionally also be referred to by the abbreviations MeO, EtO, PropO or BuO. Unless otherwise stated, the definitions propyloxy and butyloxy also include all possible isomeric forms of the groups in question. Thus, for example, propyloxy includes n-propyloxy and iso-propyloxy,

butyloxy includes iso-butyloxy, sec. butyloxy and tert. -butyloxy. The word alkoxy may also possibly be used within the scope of the present invention instead of the word alkyloxy. The groups methyloxy, ethyloxy, propyloxy or butyloxy may optionally also be referred to as methoxy, ethoxy, propoxy or butoxy.

The alkylene-alkyloxy groups used, unless otherwise stated, are branched and unbranched double-bonded alkyl bridges with 1 to 5 carbon atoms which may be mono-, di- or trisubstituted, preferably monosubstituted, by an alkyloxy group.

The -O-CO-alkyl groups used, unless otherwise stated, are branched and unbranched alkyl groups with 1 to 4 carbon atoms which are bonded via an ester group. The alkyl groups are bonded directly to the carbonylcarbon of the ester group. The term -O-CO-alkyl-halogen group should be understood analogously. The group -O-CO-$CF_3$ denotes trifluoroacetate.

Within the scope of the present invention halogen denotes fluorine, chlorine, bromine or iodine. Unless otherwise stated, fluorine and bromine are the preferred halogens. The group CO denotes a carbonyl group.

One aspect of the invention is directed to an inhalation device, in which the plural of doses are contained in one reservoir. In another aspect of the invention, the inhalation device comprises the plural of doses in a multi-dose blister pack. In another aspect of the invention the inhalation device comprises the multi-dose blister pack in form of blister strip.

The inhalation device according to the invention comprises the compounds of formula 1 preferably in admixture with a pharmaceutically acceptable excipient to form a powder mixture. The following pharmaceutically acceptable excipients may be used to prepare these inhalable powder mixtures according to the invention: monosaccharides (e.g. glucose or arabinose), disaccharides (e.g. lactose, saccharose, maltose, trehalose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose and trehalose are the particularly preferred excipients, while lactose, preferably in form of its monohydrate is most particularly preferred.

The compounds of formula 1 may be used in the form of their racemates, enantiomers or mixtures thereof. The separation of enantiomers from the racemates may be carried out using methods known in the art (e.g. by chromatography on chiral phases).

Optionally, the inhalation device according to the invention contains plural of doses of a medicament in powder form that contains, beside one compound of formula 1, another active ingredient.

Preferably the additional active ingredient is a $beta_2$ agonists 2 which is selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81 , KUL-1248, 3-(4-{6-[2-Hydroxy-2-(4-hydroxy-3- hydroxymethyl-phenyl)-ethylamino]-hexyloxy} -butyl)-benzenesulfoneamide, 5-[2-(5,6-Diethyl-indan-2-ylamino)-I-hydroxy-ethyl]-8-hydroxy-IH-quinolin-2-one, 4-hydroxy-7-[2-{[2-{[-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol , 1-[3-(4-methoxybenzyl-amino)-4-hydroxyphenyl)-2-[4-(1-benzimidazolyl)-2-methyl-2- butylaminojethanol , 1-[2H-5-hydroxy-3-oxo-4H-1,4-benzoxazin-8-yl]-2-[3-(4-N,N-dimethylaminophenyl)-2-methyl-2-propylamino]ethanol , I-[2H-5-hydroxy-3-oxo-4H-I,4-benzoxazin-8-yl]-2-[3-(4-methoxyphenyl)-2-methyl-2-propylaminojethanol, 1-[2H-5-hydroxy-3-0X0-4H-1,4-benzoxazin-8-yl]-2-[3-(4-n-butyloxyphenyl)-2-methyl-2-propylamino]ethanol , 1-[2H-5-hydroxy-3-oxo-4H-1,4-benzoxazin-8-yl]-2-{4-[3-(4-methoxyphenyl)-1,2,4-triazol-3-yl]-2-methyl-2-butylaminojethanol, 5-hydroxy-8-(1-hydroxy-2-isopropylaminobutyl)-2H-I,4-benzoxazin-3-(4H)-one, I-(4-amino-3-chloro-5-trifluormethylphenyl)-2-tert.-butylamino)ethanol and 1-(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred $beta_2$ agonists **2** are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4-{6-[2-Hydroxy-2-(4-hydroxy-3-hydroxymethyl-phenyl)-ethylamino]-hexyloxy} -butyl)-benzenesulfoneamide, 5-[2-(5,6-Diethyl-indan-2-ylamino)-I-hydroxy-ethyl]-8-hydroxy-IH-quinolin-2-one, 4-hydroxy-7-[2-{[2-{[3-(2-phenylethoxy)propyl]sulphonyl}ethyl]-amino}ethyl]-2(3H)-benzothiazolone, I-(2-fluoro-4-hydroxyphenyl)-2-[4-(I-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, I-[2H-5-hydroxy-3-oxo-4H-I,4-benzoxazin-8-yl]-2-[3-(4-N,N-dimethylaminophenyl)-2-methyl-2-propylamino]ethanol, I-[2H-5-hydroxy-3-oxo-4H-I,4-benzoxazin-8-yl]-2-[3-(4-methoxyphenyl)-2-methyl-2-propylamino]ethanol , 1-[2H-5-hydroxy-3-0X0-4H-1,4-benzoxazin-8-yl]-2-[3-(4-n-butyloxyphenyl)-2-methyl-2-propylamino]ethanol , 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(I-hydroxy-2-isopropylaminobutyl)-2H-I,4-benzoxazin-3-(4H)-one, I-(4-amino-3-chloro-5-trifluormethylphenyl)-2-tert.-butylamino)ethanol and

1-(4-ethoxycarbonylamino-3-cyano-5-fluorophenyl)-2-(tert.-butylamino)ethanol, optionally in the form of the racemates, the enantiomers, the diastereomers and optionally the pharmacologically acceptable acid addition salts and the hydrates thereof.

More preferably, the betamimetics **2** used as within the compositions according to the invention are selected from among fenoterol, formoterol, salmeterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-I-hydroxy-ethyl]-8-hydroxy- 1H-quinolin-2-one, 1-[3-(4-methoxybenzyl-amino)-4-hydroxyphenyl]-2-[4-(1-benzimidazolyl)-2-methyl-2-butylamino]ethanol , I-[2H-5-hydroxy-3-oxo-4H-I,4-benzoxazin-8-yl]-2-[3-(4-N,N-dimethylaminophenyl)-2-methyl-2-propylamino]ethanol, 1-[2H-5-hydroxy-3-oxo-4H-I,4-benzoxazin-8-yl]-2-[3-(4-methoxyphenyl)-2-methyl-2-propylamino]ethanol, I-[2H-5-hydroxy-3-oxo-4H-I,4-benzoxazin-8-yl]-2-[3-(4-n-butyloxyphenyl)-2-methyl-2-propylamino]ethanol, I-[2H-5-hydroxy-3-oxo-4H-I,4-benzoxazin-8-yl]-2-{4-[3-(4-methoxyphenyl)-1,2,4-triazol-3-yl]-2-methyl-2-butylamino}ethanol, optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethyl-phenyl)-ethylamino]-hexyloxy}-butyl)-benzenesulfoneamide, and 5-[2-(5,6-Diethyl-indan-2-ylamino)-I-hydroxy-ethyl]-8-hydroxy-1H-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics **2** according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, I-hydroxy-2-naphthalenecarboxylic acid, 4-phenylcinnamic acid, 5-(2.4-difluorophenyl)salicylic acid or maleic acid. If desired, mixtures of the abovementioned acids may also be used to prepare the salts **2.**

According to the invention, the salts of the betamimetics **2** selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of 2 in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of 2 in the case of formoterol selected from the hydrochloride, sulphate and fumarate, of which the hydrochloride and fumarate are particularly preferred. Of exceptional importance according to the invention is formoterol fumarate.

Salts of salmeterol, formoterol, 3-(4-{6-[2-Hydroxy-2-(4-hydroxy-3-hydroxymethylphenyl)-ethylamino]-hexyloxy}-butyl)-benzenesulfoneamide, and 5-[2-(5,6-Diethyl-indan-2-ylamino)-I-hydroxy-ethyl]-8-hydroxy-IH-quinolin-2-one, are preferably used as the betamimetics 2 according to the invention. Of particular importance according to the invention are salmeterol and formoterol salts. Any reference to the term betamimetics **2** also includes a reference to the relevant enantiomers or mixtures thereof. In the pharmaceutical compositions according to the invention, the compounds **2** may be present in the form of their racemates, enantiomers or mixtures thereof. The separation of the enantiomers from the racemates may be carried out using methods known in the art (e.g. by chromatography on chiral phases) If the compounds 2 are used in the form of their enantiomers, it is particularly preferable to use the enantiomers in the R configuration at the C-OH group.

Optionally, the inhalation device according to the invention contains plural of doses of a medicament in powder form, that contains beside one compound of formula 1 a steroid 3 as another active ingredient.

In such medicament combinations the steroid 3 is preferably selected from among prednisolone, prednisone , butixocortpropionate, RPR- 106541 , flunisolide , beclomethasone , triamcinolone , budesonide , fluticasone , mometasone , ciclesonide , rofleponide , ST- 126, dexamethasone , (S)-fluoromethyl 6α,9α-difluoro-17α-[(2-furanylcarbonyl)oxy]-1[beta]-hydroxy-16α-methyl-3-oxo-androsta-1,4-diene-17β-carbothionate , (S)-(2-oxo-tetrahydro-furan-3S-yl)6α,9α-difluoro-I1β-hydroxy-16α-methyl-3-oxo-17α-propionyloxy-androsta-1,4-diene-17β-carbothionate, and etiprednol- dichloroacetate (BNP- 166), optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising flunisolide , beclomethasone , triamcinolone , budesonide , fluticasone , mometasone , ciclesonide , rofleponide , ST-126, dexamethasone , (S)-fluoromethyl 6α,9α-difluoro-1Ia-[(2-furanylcarbonyl)oxy]-11β-hydroxy-16α-methyl-3-oxo-androsta-I,4-diene-17β-carbothionate, (S)-(2-oxo-tetrahydro-furan-3S-yl)6α,9α-difluoro-I Iβ-hydroxy-16α-methyl-3-oxo-17α-propionyloxy-androsta-1,4-diene-17β-carbothionate , and etiprednol-dichloroacetate , optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising budesonide, fluticasone, mometasone, ciclesonide, (S)-fluoromethyl 6α,9α-difluoro-1Ia-[(2-furanylcarbonyl)oxy]-11β-hydroxy-16α-methyl-3-oxo-androsta-1,A-diene-17β-carbothionate, and etiprednol-dichloroacetate , optionally in the form of the racemates, enantiomers or diastereomers thereof and optionally in the form of the salts and derivatives thereof, the solvates and/or hydrates thereof.

Any reference to steroids **3** includes a reference to any salts or derivatives, hydrates or solvates thereof which may exist. Examples of possible salts and derivatives of the steroids **3** may be: alkali metal salts, such as for example sodium or potassium salts, sulphobenzoates, phosphates, isonicotinates, acetates, propionates, dihydrogen phosphates, palmitates, pivalates or furcates.

Optionally, the inhalation device according to the invention contains plural of doses of a medicament on powder form, that contains beside one compound of formula **1** additionally both, one of the betamimetics **2** mentioned hereinbefore and one of the steroids **3** mentioned hereinbefore.

Accordingly, in a preferred embodiment the invention relates to an inhalation device comprising a housing and a blister strip, the strip being movable to sequentially align each blister with means for opening a blister to enable a user to inhale said dose and, a spiral wound element to receive and coil the strip, wherein each blister contains a pharmaceutical composition in powder form wherein the pharmaceutical composition comprises one or more, preferably one, compound of formula **1.**

In another embodiment, the invention relates to an inhalation device comprising a housing and a blister strip, the strip being movable to sequentially align each blister with means for opening a blister to enable a user to inhale said dose, the housing comprising a common chamber to receive the blister strip and a coil of breached blisters of that strip, the chamber being configured so that the coil of breached blisters occupies more of the space in the chamber initially occupied by the blister strip as more of the blisters of the strip are breached, wherein each blister contains a pharmaceutical composition in powder form wherein the pharmaceutical composition comprises one or more, preferably one, compound of formula **1.**

Within the scope of the inhalable powders according to the invention the excipients have a maximum average particle size of up to 250μm, preferably between 10 and 150μm, most preferably between 15 and 80μm. It may sometimes seem appropriate to add finer excipient fractions with an average particle size of 1 to 9μm to the excipients mentioned above. These finer excipients are also selected from the group of possible excipients listed hereinbefore. Finally, in order to prepare the inhalable powders according to the invention, micronised active substance 1-, and optionally 2 and/or 3, preferably with an average particle size of 0.5 to 10μm, more preferably from 1 to 6μm, is added to the excipient mixture. Processes for producing the inhalable powders according to the invention by grinding and micronising and finally mixing the ingredients together are known from the prior art.

For the methods of preparing the pharmaceutical compositions in powder form reference may be made to the disclosure of WO 02/30390, WO 03/017970, or WO 03/017979 for example.

Suitable bioactive materials also include therapeutic and prophylactic agents, such as vaccines. These include any therapeutically effective biological modifier. Such substances include subcellular compositions, cells, bacteria, viruses and molecules including lipids, organics, proteins and peptides (synthetic and natural), peptide mimetics, hormones (peptide, steroid and corticosteroid), D and L amino acid polymers, oligosaccharides, polysaccharides, nucleotides, oligonucleotides and nucleic acids, including DNA and RNA, protein nucleic acid hybrids, small molecules and physiologically active analogs thereof. Further, the modifiers may be derived from natural sources or made by recombinant or synthetic means and include analogs, agonists and homologs.

As used herein "protein" refers also to peptides and polypeptides. Such proteins include, enzymes, biopharmaceuticals, growth hormones, growth factors, insulin, antibodies, both monoclonal and polyclonal and fragments thereof, interferons, interleukins and cytokines. Organics include pharmaceutically active moieties with aromatic, carbonyl, amino, imino and guanidino groups. Suitable steroid hormones include estrogen, progesterone, testosterone and physiologically active analogs thereof. Numerous steroid hormone analogs are known in the art and include estradiol, SH-135 and tamoxifen. Many steroid hormones such as progesterone, testosterone and analogs thereof are particularly suitable for use in the present invention. As used herein, "nucleic acids" includes any therapeutically effective nucleic acids known in the art including DNA, RNA and physiologically active analogs thereof. The nucleotides may encode genes or may be any vector known in the art of recombinant DNA including plasmids, retroviruses and adeno-associated viruses. Preferably, the nucleotides are administered in the powder form of the composition.

Agents which are prophylactically active and carriers therefore are also suitable for the compositions, particles, microparticles or powders of the invention. In one embodiment, the compositions, particles, microparticles or powders comprise an immunogen such as a vaccine. Suitable vaccines include live and attenuated viruses, nucleotide vectors encoding antigens, live and attenuated bacteria, antigens, antigens plus adjuvants and haptens coupled to carriers.

Compositions containing prophylactic bioactive materials and carriers therefore are further encompassed by the invention. Preferable compositions include immunogens such as vaccines. Suitable vaccines include live and attenuated viruses, nucleotide vectors encoding antigens, bacteria, antigens, antigens plus adjuvants, haptens coupled to carriers. Particularly preferred are vaccines effective against diphtheria, tetanus, pertussis, botulinum, cholera, Dengue, Hepatitis A, C and E, hemophilus influenza b, herpes virus, Hylobacterium pylori, influenza, Japanese encephalitis,

meningococci A, B and C, measles, mumps, papilloma virus, pneumococci, polio, rubella, rotavirus, respiratory syncytial virus, Shigella, tuberculosis, yellow fever and combinations thereof.

In one embodiment of the invention, the bioactive material is hygroscopic and/or sensitive to moisture and/or labile. The bioactive material may or may not comprise an antibody and/or a human growth hormone.

In one embodiment of the invention, the bioactive material has a molecular weight or average molecular weight of less than about 22,000 Da, such as less than about 15,000 Da or less than about 10,000 Da. For example, the bioactive material may in one embodiment be proteinaceous or non-proteinaceous or a macromolecule or not a macromolecule.

In another embodiment of the invention, the bioactive material is suitable for the treatment of a respiratory disease. For example, the bioactive material may be selected from one or more of heparin, heparin sodium, immunosuppressants, such as tacrolimus, and cyclosporin as well as antifungal agents.

In one embodiment, the bioactive material comprises a therapeutic or prophylactic agent.

In another embodiment, the bioactive material is selected from a protein, enzyme, peptide, polypeptide or vaccine. The bioactive material is preferably a protein such as, for example, insulin.

The compositions of the invention may be used in medicine. For example, the compositions may be suitable for therapeutic or diagnostic use. The diagnostic use may include the use of microparticles in ultrasonic imaging, for example as echogenic contrast agents.

The composition may be used in the manufacture of a medicament for the treatment of a mammal in need thereof, such as a human. The term "mammal" also includes veterinary animals such as for example, horses, cows, sheep and pigs, as well as pets such as, for example, dogs, cats and hamsters. The condition to be treated may be one or more of asthma, chronic obstructive pulmonary disease (COPD), bronchitis, cystic fibrosis and other lung diseases, as well as acute and/or chronic lung transplant rejection and/or BO and/or BOS.

As used herein, the term "respiratory system" refers to all parts of the airway, i.e., the passageway for air during respiration, from the nose to the pulmonary alveoli. The respiratory system includes organs that are involved in breathing, such as the nose, throat, larynx, trachea, bronchi, and lungs.

As used herein, the term a "disease, disorder and/or condition of a/the respiratory system" refers to any disease, disorder and/or condition that is related to an obstructive or restrictive condition of a respiratory system. An obstructive condition of a respiratory system includes any condition which impedes the rate of air flow into and out of the lung. A restrictive condition of a respiratory system includes any condition which causes a reduction in the functional volume of the lung. The obstruction or restriction of the airway may cause symptoms such as wheezing, shortness of breath, difficulty breathing, chest tightness, and coughing. The disease, disorder and/or condition of the respiratory system can be, for example, an airway inflammatory disease, an airway stenosis, or a nasal cavity inflammatory disease.

Examples of the disease, disorder and/or condition of the respiratory system include acute lung injury (ALI); an asthma; a chronic obstructive pulmonary disease (COPD); an emphysema, a reactive airway disease (RADS); rhinitis; bronchitis; bronchiolitis; congestion; sinusitis; tonsillitis; laryngitis; post-nasal drip (PND) and any and all complications dependent on same; inflamed degranulating and non-degranulating mast cell activity; any irritation occasioning mucus secretion from goblet cells or elsewhere, resulting in breathing difficulty, restriction and/or obstruction; airway constriction or closure or mucus interference with air passage; sleep apnea; snoring; inflammatory or non-inflammatory responses to any airborne or other allergen or irritant; nasal or other airway inflammation or irritation caused by any other body area problem; physical damage to the respiratory system such as nosebleed, surgery healing, traumatic injury; any respiratory disease, disorder and/or condition caused by an airborne or seasonal allergen or irritant, or any swelling of tissue occasioned by any of the above.

As used herein, the term "asthma" refers to a chronic condition, which in most cases is characterized by reversible airway obstructions and/or constrictions. The airway becomes inflamed and is lined with excessive amounts of mucus, often in response to one or more triggers for asthma. The triggers for asthma include, but are not limited to, an environmental stimulant, such as an allergen (ragweed, house dust, animal hair, or pollen), cold air, warm air, moist air, change in temperature or humidity, upper respiratory infections, exercise, exertion, physical or emotional stress, smoke, viral illnesses such as those caused by common cold. The term "asthma" includes those caused by any cause of asthma whose primary effect is cellular inflammation and/or irritation, whether involving mast cells or not, degranulation or not, mucus exudation or not, whether exacerbant is identified or not, or whether the cause is airborne or not. The term 'asthma' is to be the widest-encompassing and is to include breathing difficulty of all degrees from the barely perceptible to acute. Examples of asthma include bronchial asthma, infantile asthma, allergic asthma, atopic asthma, steroid refractory asthma, non-allergic asthma, endogenous asthma, exogenous asthma, aspirin asthma, cardiac asthma, exercise-induced asthma, infectious asthma, any asthma triggered by airway restriction or constriction.

As used herein, the term "chronic obstructive pulmonary disease" or "COPD", also known as chronic obstructive airway disease (COAD), refers to a progressive respiratory disease characterized by limitation of airflow in the airway that is not fully reversible. COPD often involves permanent or temporary narrowing of small bronchi, in which forced expiratory flow is slowed. Examples of COPD include chronic bronchitis, emphysema and a range of other disorders to which no etiologic or other more specific term can be applied. COPD is most often due to tobacco smoking but can be

due to other airborne irritants, such as coal dust, asbestos or solvents, as well as preserved meats containing nitrites.

As used herein, the term "reactive airway disease (RAD)" refers to an asthma-like syndrome developed after a single exposure to high levels of a trigger, such as irritating vapor, fume, or smoke. In a particular embodiment of the present invention, the term RAD includes an asthma-like syndrome in infants that may later be confirmed to be asthma when they become old enough to participate in diagnostic tests.

The compositions of the invention, for example those comprising a saccharide such as heparin sodium, may be used in the treatment of one or more of the following conditions: adult respiratory distress syndrome; allergic encephalomyelitis; allergic rhinitis; arthritis; asthma; cancer; delayed type hypersensitivity reactions; inflammatory bowel disease; interstitial cystitis; respiratory disorder or disease which comprises increased levels and/or viscosity of mucus or other pulmonary secretions, such as CAL, pneumonia, sinusitis, sinus congestion, cystic fibrosis and asthma, where the subject to be treated may have a respiratory tract infection, such as a bacterial or viral infection, for example, influenza or a cold; chronic airflow limitation (CAL) with mucus hypersecretion; a disorder characterized by the presence of endogenous extracellular DNA, such as cystic fibrosis, CAL, pneumonia or systemic lupus erythematosus (SLE); transplant rejection; and pulmonary arterial hypertension (PAH).

The compositions of the invention can be used for facilitating the clearance of mucus from the central and peripheral airways of a human subject with chronic airflow limitation (CAL) who has mucus hypersecretion.

The compositions may also be used for the treatment of a pulmonary disease, such as, for example, a pulmonary disease involving hypersecretion of mucus or abnormal viscoelasticity of mucus.

In one embodiment, the pulmonary disease is selected from one or more of chronic bronchitis, acute asthma, cystic fibrosis (CF), chronic obstructive pulmonary disease (COPD) or bronchiectasis.

The compositions of the invention may be used for the treatment of the following conditions: as an anticoagulant, preventing the formation of clots and extension of existing clots within the blood; for anticoagulation for the following conditions: acute coronary syndrome, e.g., NSTEMI atrial fibrillation, deep-vein thrombosis and pulmonary embolism, cardiopulmonary bypass for heart surgery.

The compositions of the invention comprising heparin and its derivatives (enoxaparin or dalteparin) may be effective at preventing deep-vein thromboses and pulmonary emboli in patients at risk.

In one embodiment, there is provided a dry powder inhaler comprising a composition of the invention as defined in any of the embodiments herein.

The compositions, foams, powders or particles of the invention may be provided as a unit dose form. The composition, foam, powder or particles may, for example, be provided in a container. In one embodiment, a container is provided comprising the powder or microparticles according to the invention. The container may be a capsule, blister, reservoir or other receptacle for housing the powder. The compositions of the invention are particularly suitable for use in reservoir based or other multidose dry powder inhalers.

In the method of the invention, the particulate composition comprises particles which have a median geometric diameter of less than about 10$\mu$m. The particulate composition is also, for example, for administration by inhalation.

In one embodiment, the composition according to the invention is prepared by a method which comprises the step of providing a bioactive material and the metal ion salt in a solution or dispersion and spray-drying. The solution or dispersion is preferably an aqueous solution or dispersion. Typically the composition does not comprise a liquid which is more volatile than water, such as ethanol. In one embodiment, the composition, i.e., solution or dispersion, may comprise a volatile solvent, such as a solvent or liquid which is more volatile than water, such as ethanol.

Suitable inlet temperatures for the spray-drying are, for example, from 90 to 180°C, such as from 1 10°C t0 140°C, for example about 130°C. Outlet temperatures may range from, for example, 40 to 100°C, such as from 60 to 90°C, for example about 80 to 90°C.

The atomisation pressure may be from, for example 1 to 5 bar g, such as 2 to 4 bar g or about 3 bar g. In one embodiment of the invention, the inlet temperature is from about 90 to 180°C, the outlet temperature is from about 40 to 100°C and the atomisation pressure is from 1 to 5 bar g.

The weight ratio of the bioactive material to the metal ion salt used in the solution or dispersion may be, for example, from 70:30 to 99:1, for example from 80:20 to 98:2, 90:10 to 98:2, or 95:5 to 98:2.

The listing or discussion of an apparently prior-published document in this specification should not necessarily be taken as an acknowledgement that the document is part of the state of the art or is common general knowledge.

In the examples and throughout this specification, all percentages, parts and ratios are by weight unless indicated otherwise. Average molecular weights are based on weight unless otherwise specified. It will be appreciated that the various percentage amounts of the different components that are present in the products of the invention, including any optional components, will add up to 100%.

## EXAMPLES

### Reference Example 1:

### Trehalose:insulin feedstocks

An 80:20% w/w trehalose:insulin and a 20:80% w/w feedstock was prepared and spray dried. Insulin was dissolved in 0.01 M HCl. Trehalose was dissolved separately in deionised water and the solutions were combined.

The insulin was obtained from Biocon. The spray-drying conditions used for the 80:20 feedstock were as follows.

Spray-Drying Conditions

**Feed Material**

| Material / Type | Concentration (% w/v) | Volume (ml) | Mass Spray Dried (g) | Source / Batch Number | Additions |
|---|---|---|---|---|---|
| insulin | 2 | 500 | 10 | EM/05/403 | trehalose |

**Feed Details**

| Pump Setting /Tube Details | Feed Time / Masses | Feed Rate (g/min) |
|---|---|---|
| 75/1.6mm ID | $\frac{140.0 - 115.5}{5}$ | 4.9g/min |

**Drying Conditions**

| Inlet Temperature (°C) | Outlet Temperature (°C) | | Atomisation Type | Atomisation Pressure (bar g) | Atomisation Airflow (l/s) |
|---|---|---|---|---|---|
| | START | FINISH | | | |
| 130 | 84.1°C | 87.0°C | 2FN | 3.0 | 22.5 |
| | | | | Drying Air Pressure (bar g) | Dry Air Flow (l/s) |
| | | | | 2.0 | 7.5 |

The powder obtained was off white in colour. The powder was collected in low humidity conditions (-25% RH).

### Example 2:

### Calcium lactate:insulin feedstocks

Calcium lactate is obtained as a pentahydrate from PURAC (Illinois, USA) under the trade name PURACAL®.

An 80:20% w/w (calcium lactate:insulin) feedstock and a 20:80 (calcium lactate:insulin) feedstock was prepared using calcium lactate pentahydrate and insulin. Insulin was dissolved in 0.01M HCl. Calcium lactate pentahydrate was dissolved separately in deionised water and the solutions were combined. Insulin immediately precipitated out. 5M HCl was added dropwise until the solution cleared.

Spray-Drying Conditions

The spray-drying conditions used were as follows.

**Feed Material**

| Material / Type | Concentration (% w/v) | Volume (ml) | Mass Spray Dried (g) | Source / Batch Number | Additions |
|---|---|---|---|---|---|
| insulin | 2 | 500 | 10 | EM/05/403 | Calcium lactate |

**Feed Details**

| Pump Setting / Tube Details | Feed Time / Masses | Feed Rate (g/min) |
|---|---|---|
| 79/1.6mm ID | $\frac{150.0 - 124.5}{5}$ | 5.1g/min |

**Drying Conditions**

| Inlet Temperature (°C) | Outlet Temperature (°C) | | Atomisation Type | Atomisation Pressure (bar g) | Atomisation Airflow (l/s) |
|---|---|---|---|---|---|
| | START | FINISH | | | |
| 130 | 87.0°C | 89.1°C | 2FN | 3.0 | 22.5 |
| | | | | Drying Air Pressure (bar g) | Dry Air Flow (l/s) |
| | | | | 2.0 | 7.5 |

The powder was off white in colour for both feedstocks and collected in low humidity conditions (-25% RH).

## Reference Example 3:

Stability Study - Effect of Trehalose and Calcium Lactate

The 80:20 powders prepared from Examples 1 and 2 were tested.
The following tests were performed:

- Particle size distribution (geometric) using the Sympatec laser diffraction system with an R2 lens.
- The glass transition temperature (Tg) was measured using the Perkin Elmer Diamond DSC (Differential Scanning Calorimeter)

These two batches were put into open glass Petri dishes in the 25°C/60% RH stability cabinet. Regular samples were taken and analysed for particle size distribution using the Sympatec with R2 lens.
Fresh samples were placed in open glass Petri dishes in the 40°C/75% RH stability cabinet.
All samples were sealed in screw top jars with additional parafilm protection and stored in controlled low humidity conditions for any further analysis.
The results are set out in the following Tables:

Table 1

| ***Samples stored at 40°C/75% RH*** 80:20% w/w trehalose:insulin | | | | |
|---|---|---|---|---|
| | T=0 hours | T=0 hours | T=0.5 hours | T=0.5 hours |
| Batch | Individual Results | Mean | Individual Results | Mean |
| $X_{10}$ (μm) | 0.67<br>0.67<br>0.64 | 0.66 | n/a<br>n/a<br>n/a | n/a |
| $X_{50}$ (μm) | 1.50<br>1.49<br>1.48 | 1.49 | n/a<br>n/a<br>n/a | n/a |
| $X_{90}$ (μm) | 3.14<br>3.05<br>3.04 | 3.08 | n/a<br>n/a<br>n/a | n/a |
| $X_{99}$ (μm) | 5.06<br>5.11<br>5.16 | 5.11 | n/a<br>n/a<br>n/a | n/a |

No samples analysed at 0.5 hours for 40°C/75% RH. Sample appeared as fused thick paste that could not be analysed.

Table 2

| 80:20% w/w trehalose:insulin ***Samples stored at 25°C/60% RH*** | | | | |
|---|---|---|---|---|
| | T=0 hours | T=0 hours | T=16 hours | T=16 hours |
| Batch | Individual Results | Mean | Individual Results | Mean |
| $X_{10}$ (μm) | 0.67<br>0.67<br>0.64 | 0.66 | n/a<br>n/a<br>n/a | n/a |
| $X_{50}$ (μm) | 1.50<br>1.49<br>1.48 | 1.49 | n/a<br>n/a<br>n/a | n/a |
| $X_{90}$ (μm) | 3.14<br>3.05<br>3.04 | 3.08 | n/a<br>n/a<br>n/a | n/a |
| $X_{99}$ (μm) | 5.06<br>5.11<br>5.16 | 5.11 | n/a<br>n/a<br>n/a | n/a |

No samples analysed at 16 hours for 25°C/60% RH. Sample appeared as fused thick paste that could not be analysed.

Table 3

| 80:20% w/w calcium lactate:insulin ***Samples stored at 25°C/60% RH*** | | | | | | | | | | |
|---|---|---|---|---|---|---|---|---|---|---|
| | T=0 hours | T=0 hours | T=16 hours | T=16 hours | T=18 hours | T=18 hours | T=20 hours | T=20 hours | T=22 hours | T=22 hours |
| Batch | Individual Results | Mean | Individual Results | Mean | Individual Results | Mean | Individual Results | Mean | Individual Results | Mean |
| $X_{10}$ (μm) | 0.63<br>0.61<br>0.62 | 0.62 | 0.65<br>0.65<br>0.64 | 0.65 | 0.67<br>0.65<br>0.69 | 0.67 | 0.69<br>0.68<br>0.68 | 0.68 | 0.71<br>0.69<br>0.69 | 0.70 |
| $X_{50}$ (μm) | 1.49<br>1.47<br>1.48 | 1.48 | 1.55<br>1.55<br>1.56 | 1.55 | 1.59<br>1.55<br>1.60 | 1.58 | 1.60<br>1.58<br>1.59 | 1.59 | 1.64<br>1.62<br>1.61 | 1.62 |
| $X_{90}$ (μm) | 3.12<br>3.05<br>3.09 | 3.09 | 3.25<br>3.28<br>3.29 | 3.27 | 3.32<br>3.27<br>3.32 | 3.30 | 3.31<br>3.28<br>3.29 | 3.29 | 3.34<br>3.34<br>3.33 | 3.34 |
| $X_{99}$ (μm) | 4.61<br>4.68<br>4.64 | 4.64 | 4.83<br>4.85<br>4.86 | 4.85 | 4.83<br>4.92<br>4.81 | 4.85 | 4.81<br>4.84<br>4.78 | 4.81 | 4.83<br>4.83<br>4.80 | 4.82 |

There is no significant difference in particle size distribution for samples stored at 25°C/60% RH for 22 hours.

Table 4

| 80:20% w/w calcium lactate: insulin<br>***Samples stored at 25°C/60% RH*** | | | | | | | | |
|---|---|---|---|---|---|---|---|---|
| | T=0 hours | T= 0 hours | T=40 hours | T=40 hours | T=44 hours | T=44 hours | T=47 hours | T=47 hours |
| Batch | Individual Results | Mean | Individual Results | Mean | Individual Results | Mean | Individual Results | Mean |
| | 0.63 | | 0.76 | | 0.75 | | 0.79 | |
| $X_{10}$ (μm) | 0.61 | 0.62 | 0.71 | 0.74 | 0.77 | 0.76 | 0.74 | 0.76 |
| | 0.62 | | 0.75 | | 0.76 | | 0.75 | |
| | 1.49 | | 1.78 | | 1.82 | | 1.87 | |
| $X_{50}$ (μm) | 1.47 | 1.48 | 1.77 | 1.78 | 1.82 | 1.81 | 1.86 | 1.86 |
| | 1.48 | | 1.79 | | 1.79 | | 1.84 | |
| | 3.12 | | 3.66 | | 4.27 | | 3.90 | |
| $X_{90}$ (μm) | 3.05 | 3.09 | 4.30 | 4.00 | 3.93 | 4.00 | 4.47 | 4.18 |
| | 3.09 | | 4.05 | | 3.79 | | 4.17 | |
| | 4.61 | | 71.16 | | 80.13 | | 77.38 | |
| $X_{99}$ (μm) | 4.68 | 4.64 | 81.18 | 77.40 | 77.28 | 77.93 | 81.66 | 79.66 |
| | 4.64 | | 79.87 | | 76.37 | | 79.95 | |

After 40 hours at 25°C/60% RH, there are the first signs of agglomeration seen in the $X_{99}$ however even after 93 hours, the powder remained highly flowable and the $X_{50}$ particle size has only increased by 0.5μm from T0.

No further testing was performed after 93 hours.

Table 5

| 80:20% w/w calcium lactate:insulin<br>***Samples stored at 40°C/75% RH*** | | | | | | | | |
|---|---|---|---|---|---|---|---|---|
| | T=0 hours | T=0 hours | T=0.5 hours | T=0.5 hours | T=1 hour | T=1 hour | T=1.5 hours | T=1.5 hours |
| Batch | Individual Results | Mean | Individual Results | Mean | Individual Results | Mean | Individual Results | Mean |
| | 0.63 | | 0.63 | | 0.76 | | 0.95 | |
| $X_{10}$ (μm) | 0.61 | 0.62 | 0.63 | 0.64 | 0.76 | 0.76 | 0.97 | 0.96 |
| | 0.62 | | 0.66 | | 0.76 | | n/a | |
| | 1.49 | | 1.52 | | 1.79 | | 2.76 | |
| $X_{50}$ (μm) | 1.47 | 1.48 | 1.52 | 1.53 | 1.84 | 1.82 | 2.91 | 2.84 |
| | 1.48 | | 1.54 | | 1.82 | | n/a | |

(continued)

| 80:20% w/w calcium lactate:insulin *Samples stored at 40°C/75% RH* | | | | | | | | |
|---|---|---|---|---|---|---|---|---|
| | T=0 hours | T=0 hours | T=0.5 hours | T=0.5 hours | T=1 hour | T=1 hour | T=1.5 hours | T=1.5 hours |
| Batch | Individual Results | Mean | Individual Results | Mean | Individual Results | Mean | Individual Results | Mean |
| $X_{90}$ (μm) | 3.12 | 3.09 | 3.21 | 3.22 | 3.88 | 4.12 | 66.51 | 68.21 |
| | 3.05 | | 3.22 | | 4.34 | | 69.90 | |
| | 3.09 | | 3.24 | | 4.13 | | n/a | |
| $X_{99}$ (μm) | 4.61 | 4.64 | 4.81 | 4.76 | 76.07 | 78.06 | 84.92 | 85.24 |
| | 4.68 | | 4.73 | | 79.34 | | 85.56 | |
| | 4.64 | | 4.75 | | 78.78 | | n/a | |

There is no significant difference after 30 minutes at 40°C/75% RH in particle size distribution. After 1 hour, there are signs of agglomeration at the $X_{99}$ and after 1.5 hours, this has progressed to 68.2μm at $X_{90}$. The $X_{50}$ value still remains relatively low.

No samples analysed at 2 hours for 40°C/75% RH. Sample appeared as fused glass layer that could not be analysed.

Duplicate tests performed at 1.5 hours due to lack of available material. Most material in the petri dish was fused to the glass.

The results of the stability experiments for the calcium lactate formulation are shown in Figures 4 and 5.

Table 6

| | T0 hours | T1 hour 40/75 | |
|---|---|---|---|
| Components | Loss on Drying 30 - 150°C (%) | Loss in Drying 30 - 150°C (%) | Difference |
| 80:20% w/w calcium lactate:insulin | | 11.916 | 11.916 |
| | | | 0 |
| | | | 0 |
| | | | 0 |

Reference Example 4:

Differential Scanning Calorimetry

Differential Scanning Calorimetry (DSC) was performed on the following powders from Examples 1 and 2.

80:20% w/w trehalose:insulin (see Figure 1)

80:20% w/w calcium lactate:insulin (see Figure 3)

The DSC scanning rates for Figure 1 and Figure 3 were: 1) hold for 1.0 minute at 0.00°C; 2) heat from 0.00°C to 250.00°C at 50.00°C /min; 3) hold for 1.0 min at 250.00°C.

The scanning rate for Figure 2 was: 1) heat from 25.00°C to 200.00°C at 10.00°C/min; 2) hold for 1.0 minute at 200.00°C; 3) Cool from 200.00°C to 0.00°C at 40.00°C/min 1; 4) hold for 1.0 minute at 0.00°C; 5) heat from 0.00°C to 200.00°C at 10.00°C /min

Both powders were tested at the TO timepoint. The 80% trehalose batch has a Tg of 82°C whereas the calcium lactate batch has a far higher Tg of 121°C.

Both powders were then placed in 25°C/60% RH condition in open Petri dishes overnight. After 16 hours, the trehalose containing batch had fused into a thick paste that could not be analysed. The calcium lactate batch remained as a free flowing powder and was left in the stability cabinet at 25°C/60% RH for 93 hours. DSC analysis was performed again and also at the mid-point (44hours).

44 hours at 25°/60% RH = Tg of 87°C
93 hours at 25°/60% RH = Tg of 87°C

Even at stressed conditions after 93 hours, the Tg of the calcium lactate sample remained higher than the trehalose batch at TO (unstressed). The particle size data shows how the particle size distribution remained relatively consistent for the calcium lactate sample after 93 hours and it remained a white free flowing powder.

Summary

DSC data generated indicates a Tg of about 80°C for trehalose:insulin (80:20) at about 4% residual moisture content.

DSC data generated indicates a Tg of 122°C for calcium lactate:insulin (80:20) at about 3.5% residual moisture content, with a large $\Delta C_p$ of about 2.652.

At 25°C/60% RH, the calcium lactate composition is still stable and is free-flowing. Under the same conditions, the trehalose composition fuses and has the appearance of a "molten globule".

At 40°C/75%RH, the calcium lactate powder still has a size of 1.5 microns after 30 minutes. After 1 hour, the size was 1.84 microns and after 1.30 hours, the size was 2.81 microns. Under the same conditions, the trehalose composition fuses.

Reference Example 5:

Assessment of the Stabilising Properties of Salts

**Objective**

To assess the stabilising properties of calcium lactate, sodium lactate and sodium citrate salts in spray dried formulations containing tiotropium bromide.

**Method**

Spray dried formulations were prepared as described above. The formulations were prepared at a tiotropium bromide / stabiliser ratio of 20/80. The formulations were subjected to environmental conditions of 40°C and 75% relative humidity for 24 h. A tiotropium / trehalose formulation was also assessed to enable a comparison of the stabilising properties of salts with sugars. Samples were taken at initial and at subsequent 2 h intervals and subjected to particle size distribution (PSD) analysis using the TSI®-aerodynamic particle size analyser (TSI-APS). The TSI-APS was set up with the following default parameters:
Particle density = 1.00 g/cc; side scatter data types = channel data and raw data selected; correlated sample mode selected; high concentration threshold set to 5000 #/cc.

The particle size data is based on the number of particle counts per size channel per unit of volume of air sampled

Each sample was measured in duplicate. The calculated mean particle size and the geometric standard deviation from each measurement are presented in Table 7.

Table 7: Summary of TSI-APS Mean Particle Size Distribution and Mean Geometric Standard Deviation Data

| **Formulation Description and Batch Number** | **Timepoint (h)** | **Mean PSD (μm)** | | **Geometric Standard Deviation** | |
|---|---|---|---|---|---|
| | | **1** | **2** | **1** | **2** |
| Tiotropium / trehalose RTB100720APA | Initial | 3.35 | 3.80 | 2.07 | 1.99 |
| | 2 | 3.33 | 3.38 | 1.84 | 1.83 |
| Tiotropium / calcium lactate RTB100722MKA | Initial | 2.93 | 3.15 | 1.68 | 1.68 |
| | 2 | 2.86 | 2.85 | 1.64 | 1.66 |
| | 4 | 2.97 | 2.89 | 1.72 | 1.71 |
| | 6 | 3.02 | 3.19 | 1.68 | 1.68 |

(continued)

| Formulation Description and Batch Number | Timepoint (h) | Mean PSD (μm) | | Geometric Standard Deviation | |
|---|---|---|---|---|---|
| | | 1 | 2 | 1 | 2 |
| Tiotropium / sodium citrate RTB101012APB | Initial | 3.25 | 2.94 | 1.71 | 1.67 |
| | 2 | 3.57 | 3.15 | 1.76 | 1.71 |
| | 4 | 3.77 | 4.16 | 1.70 | 1.71 |
| Tiotropium / sodium lactate RTB101012APA | Initial | 1.96 | N/A | N/A | N/A |

At the 4 h timepoint tiotropium / trehalose sample had formed large, solid particles which were too large to pass through the TSI-APS inlet aperture thus no further analysis was possible.

The tiotropium / calcium lactate sample retained its particle size past the 6 h timepoint. By the 24 h timepoint it had formed large solid particles which were unsuitable for analysis by TSI-APS.

At the 6 h timepoint the tiotropium / sodium citrate sample had formed large solid particles, unsuitable for analysis using the TSI-APS.

The tiotropium / sodium lactate sample was difficult to process. The initial sample formed one large sticky agglomerate which stuck to the bristles of the brush used to add samples to the TSI-APS inlet. An initial sample was processed but the data was considered to be anomalous and not a true reflection of the particle size of the material. The further samples taken at the 2 h and 4 h timepoints were not only sticky but also looked wet indicative on the deliquescent nature of the salt.

The data shows that the salts can be ranked in order of their ability to resist moisture uptake. Calcium lactate has the best moisture resistance properties with a change in appearance from friable agglomerates to solid agglomerates occurring some time after 6h but less than 24 h. To enable a clearer indication of the moisture resistance properties of calcium lactate the comparison between calcium lactate and sodium citrate was repeated. The results from the repeat test are presented in Table 8.

Table 8: Summary of TSI-APS Mean Particle Size Distribution and Mean Geometric Standard Deviation Data

| Formulation Description and Batch Number | Timepoint (h) | Mean PSD (μm) | | Geometric Standard Deviation | |
|---|---|---|---|---|---|
| | | 1 | 2 | 1 | 2 |
| Tiotropium / calcium lactate RTB100722MKA | Initial | 3.45 | 3.24 | 1.62 | 1.69 |
| | 2 | 3.37 | 3.58 | 1.68 | 1.69 |
| | 3 | 3.08 | 3.23 | 1.69 | 1.70 |
| | 4 | 3.13 | 3.11 | 1.66 | 1.67 |
| | 6 | 3.05 | 3.23 | 1.61 | 1.64 |
| | 7 | 2.89 | 2.96 | 1.58 | 1.60 |
| | 8 | 3.06 | 3.13 | 1.58 | 1.57 |
| | 9 | 3.11 | 3.02 | 1.59 | 1.56 |
| | 24 | N/A | N/A | N/A | N/A |
| Tiotropium / sodium citrate RTB101012APB | Initial | 3.27 | 3.85 | 1.75 | 1.75 |
| | 2 | 3.54 | 3.79 | 1.76 | 1.78 |
| | 3 | 3.23 | 3.38 | 1.81 | 1.77 |
| | 4 | 3.10 | 3.03 | 1.77 | 1.80 |
| | 5 | N/A | N/A | N/A | N/A |

At 5h the agglomerates in the tiotropium / sodium citrate sample had solidified.

At the 8h timepoint the tiotropium / calcium lactate sample contained some friable agglomerates.
At the 9 h timepoint the agglomerates appeared less friable. The agglomerates in the sample had solidified at 24h.

Example 6:

To evaluate the moisture protection of APIs for spray dried formulations containing calcium lactate, batches of formulations were prepared using calcium lactate in combination with different APIs and excipients. The formulations of this example which contain trehalose in an amount of 40% are not in the scope of the claims.

**Fluticasone Propionate (FP)**

The first phase of work looked at the manufacture of spray dried batches containing FP at the following proportions:

100% FP
50/40/10% FP/trehalose/calcium lactate
50/40/10% FP/trehalose/sodium citrate

All three blends were placed in a stability cabinet set at 25°C/60% RH, with sub samples taken for analysis after 2, 4, 6 and 24 hours, then after 2 and 5 days.
Analysis was performed using the TSI - aerodynamic particle sizer (APS) to measure the aerodynamic particle size distribution. An increase in particle size would be expected for samples without an excipient to protect the API from moisture absorption.
APS parameters: Particle density = 1.00 g/cc; side scatter data types = channel data and raw data selected; correlated sample mode selected; high concentration threshold set to 5000 #/cc. Samples analysed in duplicate.
Table 9 provides a summary of the measured aPSD data obtained from each timepoint.

**Table 9: Summary of aPSD Data for Fluticasone Propionate Blends**

| **Blend** | **Timepoint** | **Mean aPSD (µm)** | | **Geometric Standard Deviation** | |
|---|---|---|---|---|---|
| | | 1 | 2 | 1 | 2 |
| **100% API** | Initial | 2.83 | 2.64 | 1.58 | 1.56 |
| | 2 hours | 2.47 | 2.3 | 1.57 | 1.53 |
| | 4 hours | 2.39 | 2.66 | 1.52 | 1.57 |
| | 6 hours | 2.6 | 2.97 | 1.58 | 1.65 |
| | 24 hours | 2.89 | 2.75 | 1.63 | 1.59 |
| | 2 days | 1.83 | 2.05 | 1.80 | 1.84 |
| | 5 hrs | 2.16 | 2.61 | 1.57 | 1.61 |
| **50/40/10 (w/w) FP / trehalose / calcium lactate** | Initial | 2.41 | 2.33 | 1.58 | 1.59 |
| | 2 hours | 2.23 | 2.59 | 1.57 | 1.59 |
| | 4 hours | 2.49 | 2.63 | 1.57 | 1.60 |
| | 6 hours | 2.45 | 2.4 | 1.74 | 1.77 |
| | 24 hours | 2.67 | 2.68 | 1.60 | 1.59 |
| | 2 days | 1.92 | 2.01 | 1.89 | 1.86 |
| | 5 hrs | 2.75 | 2.47 | 1.64 | 1.57 |

(continued)

| Blend | Timepoint | Mean aPSD (μm) | | Geometric Standard Deviation | |
|---|---|---|---|---|---|
| | | 1 | 2 | 1 | 2 |
| **50/40/10 (w/w) API / trehalose /sodium citrate** | Initial | 2.59 | 2.31 | 1.71 | 1.70 |
| | 2 hours | 2.15 | 2.17 | 1.61 | 1.55 |
| | 4 hours | 2.29 | 2.09 | 1.57 | 1.54 |
| | 6 hours | 2.23 | 2.54 | 1.70 | 1.82 |
| | 24 hours | 2.15 | 2.55 | 1.58 | 1.59 |
| | 2 days | 2.01 | 1.92 | 1.92 | 1.85 |
| | 5 hrs | 2.32 | 2.59 | 1.59 | 1.57 |

After five days there was no discernable change in particle size measured by the APS. It was concluded that FP was an unsuitable API to use to model the effects of a moisture absorbing excipient, due to the very low natural water solubility of FP.

**Salbutamol Sulphate**

Salbutamol sulphate was then selected due to its good water solubility making it a suitable candidate to model the effects of calcium lactate when blended with the API. The following blends were spray dried:

100% salbutamol sulphate
90/10 (w/w) salbutamol sulphate / calcium lactate
50/40/10 (w/w) salbutamol sulphate / trehalose / calcium lactate

All three blends were placed in a stability cabinet set at 25°C / 60% RH, with sub samples taken for analysis after 2, 4, 6 and 24 hours, then after 5 and 6 days. Samples then transferred to the 40°C / 75% RH stability cabinet after the 6 day timepoint and then samples taken for analysis after 24 and 48 hrs.

Analysis performed using the TSI-APS with conditions as before. See Table 10.

**Table 10: Summary of aPSD Data for Salbutamol Sulphate Blends**

| Blend | Timepoint | Mean aPSD (μm) | | Geometric Standard Deviation | |
|---|---|---|---|---|---|
| | | 1 | 2 | 1 | 2 |
| 100% API | Initial | 2.72 | 2.73 | 1.68 | 1.72 |
| | 2 hours | 2.27 | 2.85 | 1.66 | 1.74 |
| | 4 hours | 2.42 | 2.62 | 1.74 | 1.69 |
| | 6 hours | 2.74 | 2.31 | 1.79 | 1.72 |
| | 24 hours | 2.44 | 2.28 | 1.74 | 1.71 |
| | 5 days | 2.47 | 2.87 | 1.68 | 1.72 |
| | 6 days | 2.37 | 2.6 | 1.67 | 1.67 |
| | 24 hrs (40/75) | n/a | n/a | n/a | n/a |
| | 48 hrs (40/75) | n/a | n/a | n/a | n/a |

(continued)

| Blend | Timepoint | **Mean aPSD (μm)** | | **Geometric Standard Deviation** | |
|---|---|---|---|---|---|
| | | 1 | 2 | 1 | 2 |
| 90/10 (w/w) API / calcium lactate | Initial | 2.48 | 2.71 | 1.70 | 1.69 |
| | 2 hours | 2.56 | 2.64 | 1.73 | 1.74 |
| | 4 hours | 2.35 | 2.54 | 1.72 | 1.75 |
| | 6 hours | 2.68 | 2.5 | 1.77 | 1.73 |
| | 24 hours | 2.94 | 2.62 | 1.77 | 1.73 |
| | 5 days | 2.77 | 2.84 | 1.77 | 1.83 |
| | 6 days | 2.73 | 2.63 | 1.79 | 1.76 |
| | 24 hrs (40/75) | 2.75 | 2.53 | 1.76 | 1.78 |
| | 48 hrs (40/75) | n/a | n/a | n/a | n/a |
| 50/40/10 (w/w) API / trehalose / calcium lactate | Initial | 2.37 | 2.42 | 1.72 | 1.72 |
| | 2 hours | 2.47 | 2.82 | 1.77 | 1.78 |
| | 4 hours | 2.64 | 2.68 | 1.74 | 1.73 |
| | 6 hours | 2.44 | 2.44 | 1.71 | 1.74 |
| | 24 hours | 2.67 | 2.60 | 1.75 | 1.73 |
| | 5 days | 2.77 | 3.10 | 1.81 | 1.81 |
| | 6 days | 2.94 | 2.59 | 1.74 | 1.77 |
| | 24 hrs (40/75) | 2.94 | 2.71 | 1.73 | 1.77 |
| | 48 hrs (40/75) | n/a | n/a | n/a | n/a |

After 24 hours at 40°C/75% RH the 100% API had formed large, solid crystalline particles that were too big for the aperture in the internal inlet of the TSI-APS, blocking the instrument, not allowing a reading to be taken.

After 48 hours all three samples had formed large, crystalline particles that could not be broken up with the typical sample preparation method for insertion of samples into the APS instrument. Therefore, analysis could not be performed.

However, this test demonstrated that storing the samples at the more aggressive 40°C / 75% RH was a better way to model the effects of a moisture absorbing excipient.

Two further batches, which are comparative examples, were manufactured:

90/10 (w/w) salbutamol sulphate / sodium citrate
50/40/10 (w/w) salbutamol sulphate / trehalose / sodium citrate

These were placed in a stability cabinet set at 40°C / 75% RH. Sub-samples were taken for analysis at several timepoints and measured for aPSD using the APS as described above. See Table 11

**Table 11: Summary of aPSD Data for Salbutamol Sulphate / Sodium Citrate Blends**

| Blend | Timepoint | **Mean aPSD (μm)** | | **Geometric Standard Deviation** | |
|---|---|---|---|---|---|
| | | 1 | 2 | 1 | 2 |
| 90/10 (w/w) API / sodium citrate | Initial | 3.37 | 3.37 | 1.76 | 1.76 |
| | 2 hours | 3.14 | 3.44 | 1.78 | 1.82 |
| | 3 hours | 3.11 | 3.17 | 1.76 | 1.83 |
| | 4 hours | 2.91 | 3.11 | 1.76 | 1.82 |
| | 6 hours | 3.57 | 3.63 | 1.76 | 1.72 |
| | 7 hours | n/a | n/a | n/a | n/a |
| | 24 hours | n/a | n/a | n/a | n/a |
| 50/40/10 (w/w) API / trehalose / sodium citrate | Initial | 3.22 | 3.32 | 1.69 | 1.78 |
| | 2 hours | 3.03 | 3.11 | 1.72 | 1.80 |
| | 3 hours | 2.97 | 2.83 | 1.81 | 1.75 |
| | 4 hours | 2.83 | 3.04 | 2.04 | 1.73 |
| | 6 hours | 2.78 | 2.93 | 1.73 | 1.74 |
| | 7 hours | 3.06 | 3.26 | 1.82 | 1.81 |
| | 24 hours | n/a | n/a | n/a | n/a |

At the 7 hour timepoint, the 90/10 (w/w) API / sodium citrate sample had formed large, crystalline particles, unsuitable for use in the APS, meaning their particle size could not be measured.

At the 24 hours timepoint, the 50/40/10 (w/w) API / trehalose / sodium citrate sample had formed large, crystalline particles too.

From use with salbutamol sulphate, sodium citrate is not as effective as calcium lactate in protecting the API from moisture.

## Claims

**1.** A composition for inhalation comprising a pharmaceutically acceptable glassy matrix and at least one bioactive material within the matrix, wherein the glassy matrix comprises a polyvalent metal ion salt as a glass-forming material, wherein the composition is in the form of a powder and polyols are present in an amount of less than 1 wt% based on the weight of the composition, and wherein the polyvalent metal ion salt is calcium lactate, calcium lactate citrate, or calcium lactate gluconate, wherein the powder comprises particles having a median geometric diameter of less than 10 μm and wherein the metal ion salt is present in the composition in an amount of less than or equal to 30 wt.% based on the total weight of the composition.

**2.** The composition according to Claim 1, wherein the metal ion salt is present in the composition in an amount of less than or equal to 20 wt.% based on the total weight of the composition.

**3.** The composition according to Claim 1, wherein the metal ion salt is present in the composition in an amount of from 1 to 30 wt.%, or from 5 to 30 wt.%.

**4.** The composition according to Claim 1, wherein the metal ion salt is a treated metal ion salt in an anhydrous and/or lower hydrate form, wherein the treated metal ion salt is at least a dihydrate, such as calcium lactate pentahydrate, that has been treated so as to reduce the degree of hydration.

**5.** The composition according to any one of Claims 2 to 4, wherein the composition is less hygroscopic under ambient conditions compared to compositions which do not comprise the metal ion salt or the glassy matrix consists of a polyvalent metal ion as the glass-former.

6. The composition of any one of Claims 1 to 5, wherein the bioactive is non-proteinaceous or is selected from one or more of heparin, heparin sodium, immunosuppressants, such as tacrolimus, and cyclosporin or wherein the bioactive is suitable for the treatment of a respiratory disease, such as combinations of a steroid and $\beta_2$-agonist, or tiotropium bromide.

7. The composition according to any one of Claims 1 to 6, wherein the composition is an amorphous powder, preferably a spray-dried amorphous powder or wherein the composition is for pulmonary administration.

8. The composition according to any one of Claims 1 to 7, wherein the composition consists of the glassy matrix, optionally solvent and/or water, and the at least one bioactive material, and wherein the glassy matrix is formed from the metal ion salt.

9. The composition according to any one of Claims 1 to 8, wherein the Tg of the composition and/or metal ion salt is at least about 70°C or 100°C, such as from 100 to 150°C, preferably from 110 to 130°C.

10. The composition according to any one of Claims 1 to 9, wherein the composition comprises particles having a tap density of less than or equal to about 0.4 g/cm$^3$, or the particles are hollow.

11. The composition according to any one of Claims 1 to 10, wherein the composition is in the form of a dry powder, or wherein the matrix comprises a solid solution of the at least one bioactive material.

12. The composition according to any one of Claims 1 to 11 wherein the metal ion salt is calcium lactate citrate or calcium lactate gluconate.

13. The composition according to any one of Claims 1 to 12, wherein the bioactive material comprises a therapeutic or a prophylactic agent, and/or wherein the bioactive material is selected from a protein, enzyme, peptide, polypeptide, vaccine or molecules having a molecular weight less than about 10,000 Da.

14. A method of increasing the physical or chemical stability, such as maintaining dispersibility or reducing agglomeration, of a particulate composition when subject to moisture and/or heat comprising the step of forming a composition according to any one of Claims 1 to 13.

15. The method of Claim 14 wherein the particulate composition comprises particles which have a mass median aerodynamic diameter (MMAD) of 0.1 to 10μm or, wherein the particulate composition is for administration by oral or nasal inhalation.

16. A method of producing a composition according to any one of Claims 1 to 13 which comprises the step of providing a bioactive material and a metal ion salt, such as in the form of, for example a solution, dispersion, suspension or emulsion, and spray-drying, wherein the metal ion salt is calcium lactate, calcium lactate citrate, or calcium lactate gluconate, and the composition is free of polyols.

## Patentansprüche

1. Zusammensetzung zum Inhalieren, die eine pharmazeutisch unbedenkliche glasartige Matrix und wenigstens ein bioaktives Material innerhalb der Matrix umfasst, wobei die glasartige Matrix ein polyvalentes Metallionensalz als ein glasbildendes Material umfasst, wobei die Zusammensetzung in der Form eines Pulvers ist und Polyole in einer Menge von weniger als 1 Gew.-% bezogen auf das Gewicht der Zusammensetzung vorliegen, und wobei das polyvalente Metallionensalz Calciumlactat, Calciumlactatcitrat oder Calciumlactatgluconat ist, wobei das Pulver Partikel mit einem mittleren geometrischen Durchmesser von weniger als 10 μm umfasst, und wobei das Metallionensalz in der Zusammensetzung in einer Menge von höchstens 30 Gew.-% bezogen auf das Gesamtgewicht der Zusammensetzung vorliegt.

2. Zusammensetzung nach Anspruch 1, wobei das Metallionensalz in der Zusammensetzung in einer Menge von höchstens 20 Gew.-% bezogen auf das Gesamtgewicht der Zusammensetzung vorliegt.

3. Zusammensetzung nach Anspruch 1, wobei das Metallionensalz in der Zusammensetzung in einer Menge von 1 bis 30 Gew.-% oder von 5 bis 30 Gew.-% vorliegt.

4. Zusammensetzung nach Anspruch 1, wobei das Metallionensalz ein behandeltes Metallionensalz in einer nichtwässrigen und/oder in einer niedrigeren Hydratform ist, wobei das behandelte Metallionensalz wenigstens ein Dihydrat ist, wie etwa Calciumlactatpentahydrat, das behandelt wurde, um den Grad an Hydratation zu reduzieren.

5. Zusammensetzung nach einem der Ansprüche 2 bis 4, wobei die Zusammensetzung im Vergleich zu Zusammensetzungen, die das Metallionensalz nicht umfassen, unter Umgebungsbedingungen weniger hygroskopisch ist oder die glasartige Matrix aus einem polyvalenten Metallion als der Glasbildner besteht.

6. Zusammensetzung nach einem der Ansprüche 1 bis 5, wobei der bioaktive Stoff nicht proteinhaltig ist oder aus einem oder mehreren von Heparin, Heparinnatrium, immunsuppressiven Mitteln wie etwa Tacrolimus und Cyclosporin ausgewählt ist, oder wobei der bioaktive Stoff für die Behandlung einer Atemwegserkrankung geeignet ist, wie etwa Kombinationen aus einem Steroid und einem $\beta_2$-Agonisten, oder Tiotropiumbromid.

7. Zusammensetzung nach einem der Ansprüche 1 bis 6, wobei die Zusammensetzung ein nichtkristallisches Pulver, bevorzugt ein sprühgetrocknetes nichtkristallisches Pulver ist, oder wobei die Zusammensetzung für die pulmonale Verabreichung ist.

8. Zusammensetzung nach einem der Ansprüche 1 bis 7, wobei die Zusammensetzung aus der glasartigen Matrix, optional einem Lösungsmittel und/oder Wasser und dem wenigstens einen bioaktiven Material besteht, und wobei die glasartige Matrix aus dem Metallionensalz ausgebildet ist.

9. Zusammensetzung nach einem der Ansprüche 1 bis 8, wobei die Tg der Zusammensetzung und/oder des Metallionensalzes bei wenigstens etwa 70 °C oder 100 °C, wie etwa von 100 bis 150 °C, bevorzugt von 110 bis 130 °C liegt.

10. Zusammensetzung nach einem der Ansprüche 1 bis 9, wobei die Zusammensetzung Partikel mit einer Klopfdichte von höchstens etwa 0,4 g/cm$^3$ umfasst oder die Partikel hohl sind.

11. Zusammensetzung nach einem der Ansprüche 1 bis 10, wobei die Zusammensetzung in der Form eines trockenen Pulvers ist oder wobei die Matrix eine feste Lösung des wenigstens einen bioaktiven Materials umfasst.

12. Zusammensetzung nach einem der Ansprüche 1 bis 11, wobei das Metallionensalz Calciumlactatcitrat oder Calciumlactatgluconat ist.

13. Zusammensetzung nach einem der Ansprüche 1 bis 12, wobei das bioaktive Material einen therapeutischen oder einen prophylaktischen Wirkstoff umfasst, und/oder wobei das bioaktive Material aus einem Protein, Enzym, Peptid, Polypeptid, Impfstoff oder Molekülen mit einer Molekülmasse von weniger als etwa 10.000 Da ausgewählt ist.

14. Verfahren zum Erhöhen der physikalischen oder der chemischen Stabilität, wie etwa Erhalten von Dispergierbarkeit oder Reduzieren von Agglomeration, einer speziellen Zusammensetzung, wenn sie Feuchtigkeit und/oder Wärme ausgesetzt ist, umfassend den Schritt des Ausbildens einer Zusammensetzung nach einem der Ansprüche 1 bis 13.

15. Verfahren nach Anspruch 14, wobei die spezielle Zusammensetzung Partikel mit einem medianen massebezogenen aerodynamischen Durchmesser (*mass median aerodynamic diameter* - MMAD) von 0,1 bis 10 μm umfasst, wobei die spezielle Zusammensetzung zur Verabreichung durch orale oder nasale Inhalation ist.

16. Verfahren zum Herstellen einer Zusammensetzung nach einem der Ansprüche 1 bis 13, das den Schritt des Bereitstellens eines bioaktiven Materials und eines Metallionensalzes, wie etwa in der Form beispielsweise einer Lösung, Dispersion, Suspension oder Emulsion, und Sprühtrocknen umfasst, wobei das Metallionensalz Calciumlactat, Calciumlactatcitrat oder Calciumlactatgluconat ist und die Zusammensetzung frei von Polyolen ist.

**Revendications**

1. Composition à inhaler comprenant une matrice vitreuse pharmaceutiquement acceptable et au moins un matériau bioactif dans la matrice, la matrice vitreuse comprenant un sel d'ion métallique polyvalent comme matière vitrifiable, la composition étant sous la forme d'une poudre et des polyols sont présents dans une quantité inférieure à 1 % en poids par rapport au poids de la composition, et le sel d'ion métallique polyvalent étant du lactate de calcium, du lactate-citrate de calcium ou du lactate-gluconate de calcium, la poudre comprenant des particules ayant un

diamètre géométrique moyen inférieur à 10 μm et le sel d'ion métallique étant présent dans la composition dans une quantité inférieure ou égale à 30 % en poids par rapport au poids total de la composition.

**2.** Composition selon la revendication 1, dans laquelle le sel d'ion métallique est présent dans la composition dans une quantité inférieure ou égale à 20 % en poids par rapport au poids total de la composition.

**3.** Composition selon la revendication 1, dans laquelle le sel d'ion métallique est présent dans la composition dans une quantité comprise entre 1 et 30 % en poids, ou entre 5 et 30 % en poids.

**4.** Composition selon la revendication 1, dans laquelle le sel d'ion métallique est un sel d'ion métallique traité sous une forme anhydre et/ou d'hydrate inférieur, dans laquelle le sel d'ion métallique traité est au moins un dihydrate, tel qu'un lactate-pentahydrate de calcium, qui a été traité afin de réduire le degré d'hydratation.

**5.** Composition selon l'une quelconque des revendications 2 à 4, la composition étant moins hygroscopique dans des conditions ambiantes par rapport à des compositions qui ne contiennent pas le sel d'ion métallique ou la matrice vitreuse est constituée d'un ion métallique polyvalent en tant que vitrifiant.

**6.** Composition selon l'une quelconque des revendications 1 à 5, dans laquelle le matériau bioactif est non protéique ou est choisi parmi un ou plusieurs matériaux dont l'héparine, l'héparine sodique, des immunosuppresseurs, comme le tacrolimus et la cyclosporine, ou dans laquelle le matériau bioactif est adapté au traitement d'une maladie respiratoire, comme des combinaisons d'un stéroïde et d'agoniste $\beta_2$, ou du bromure de tiotropium.

**7.** Composition selon l'une quelconque des revendications 1 à 6, la composition étant une poudre amorphe, de préférence une poudre amorphe séchée par pulvérisation ou la composition étant destinée à une administration pulmonaire.

**8.** Composition selon l'une quelconque des revendications 1 à 7, la composition étant constituée de la matrice vitreuse, éventuellement d'un solvant et/ou d'eau, et de l'au moins un matériau bioactif, la matrice vitreuse étant formée à partir du sel d'ion métallique.

**9.** Composition selon l'une quelconque des revendications 1 à 8, dans laquelle la Tg de la composition et/ou du sel d'ion métallique est au moins environ de 70 °C ou de 100 °C, par exemple de 100 à 150 °C, de préférence de 110 à 130 °C.

**10.** Composition selon l'une quelconque des revendications 1 à 9, la composition comprenant des particules ayant une densité après tassement inférieure ou égale à environ 0,4 g/cm$^3$, ou les particules sont creuses.

**11.** Composition selon l'une quelconque des revendications 1 à 10, la composition étant sous la forme d'une poudre sèche, ou la matrice comprenant une solution solide de l'au moins un matériau bioactif.

**12.** Composition selon l'une quelconque des revendications 1 à 11 dans laquelle le sel d'ion métallique est du lactate-citrate de calcium ou du lactate-gluconate de calcium.

**13.** Composition selon l'une quelconque des revendications 1 à 12, dans laquelle le matériau bioactif comprend un agent thérapeutique ou prophylactique et/ou dans laquelle le matériau bioactif est choisi parmi une protéine, une enzyme, un peptide, un polypeptide, un vaccin ou des molécules ayant une masse moléculaire inférieure à environ 10 000 Da.

**14.** Procédé d'augmentation de la stabilité physique ou chimique, comme le maintien de la dispersibilité ou la réduction de l'agglomération, d'une composition particulaire lorsqu'elle est soumise à l'humidité et/ou à la chaleur comprenant l'étape de formation d'une composition selon l'une quelconque des revendications 1 à 13.

**15.** Procédé selon la revendication 14 dans lequel la composition particulaire comprend des particules qui ont un diamètre aérodynamique moyen en masse (DAMM) de 0,1 à 10 μm ou, dans lequel la composition particulaire est destinée à une administration par inhalation orale ou nasale.

**16.** Procédé de production d'une composition selon l'une quelconque des revendications 1 à 13 qui comprend l'étape de fourniture d'un matériau bioactif et d'un sel d'ion métallique, par exemple sous la forme d'une solution, d'une

dispersion, d’une suspension ou d’une émulsion, et de séchage par pulvérisation, le sel d’ion métallique étant du lactate de calcium, du lactate-citrate de calcium ou du lactate-gluconate de calcium et la composition est exempte de polyols.

Figure 1

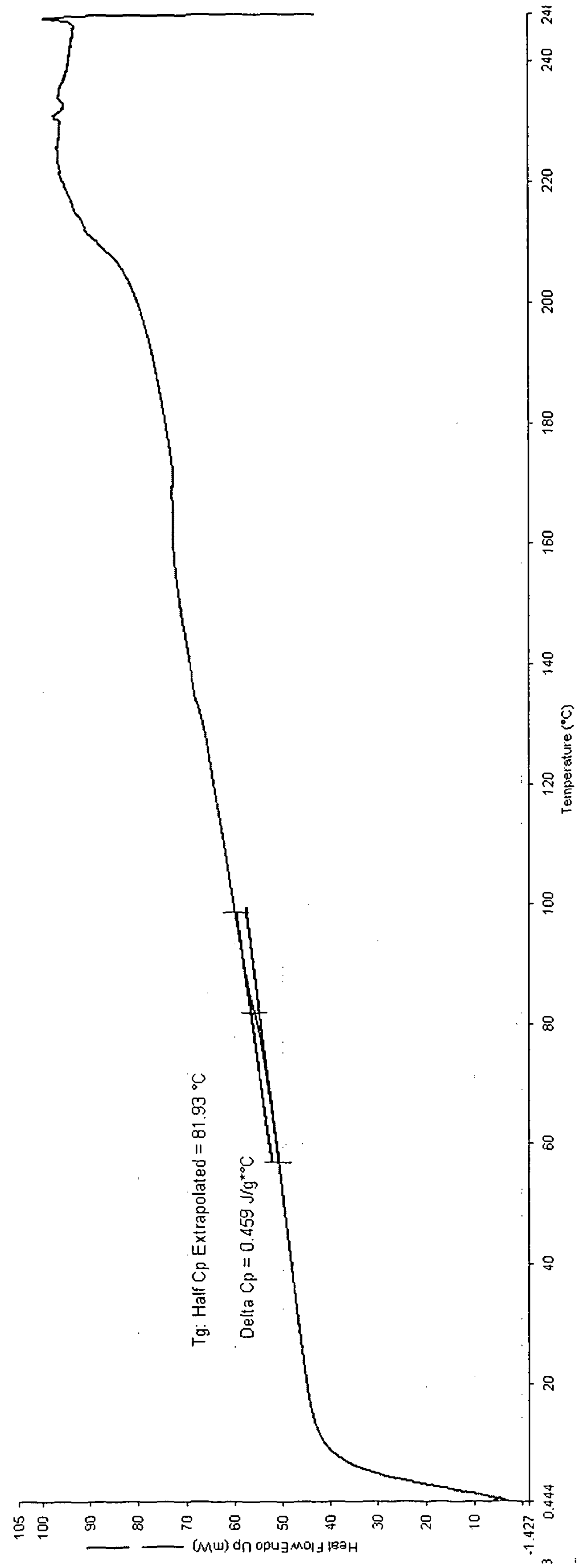
Tg: Half Cp Extrapolated = 81.93 °C
Delta Cp = 0.459 J/g*°C
Heat Flow Endo Up (mW)
Temperature (°C)

Figure 2

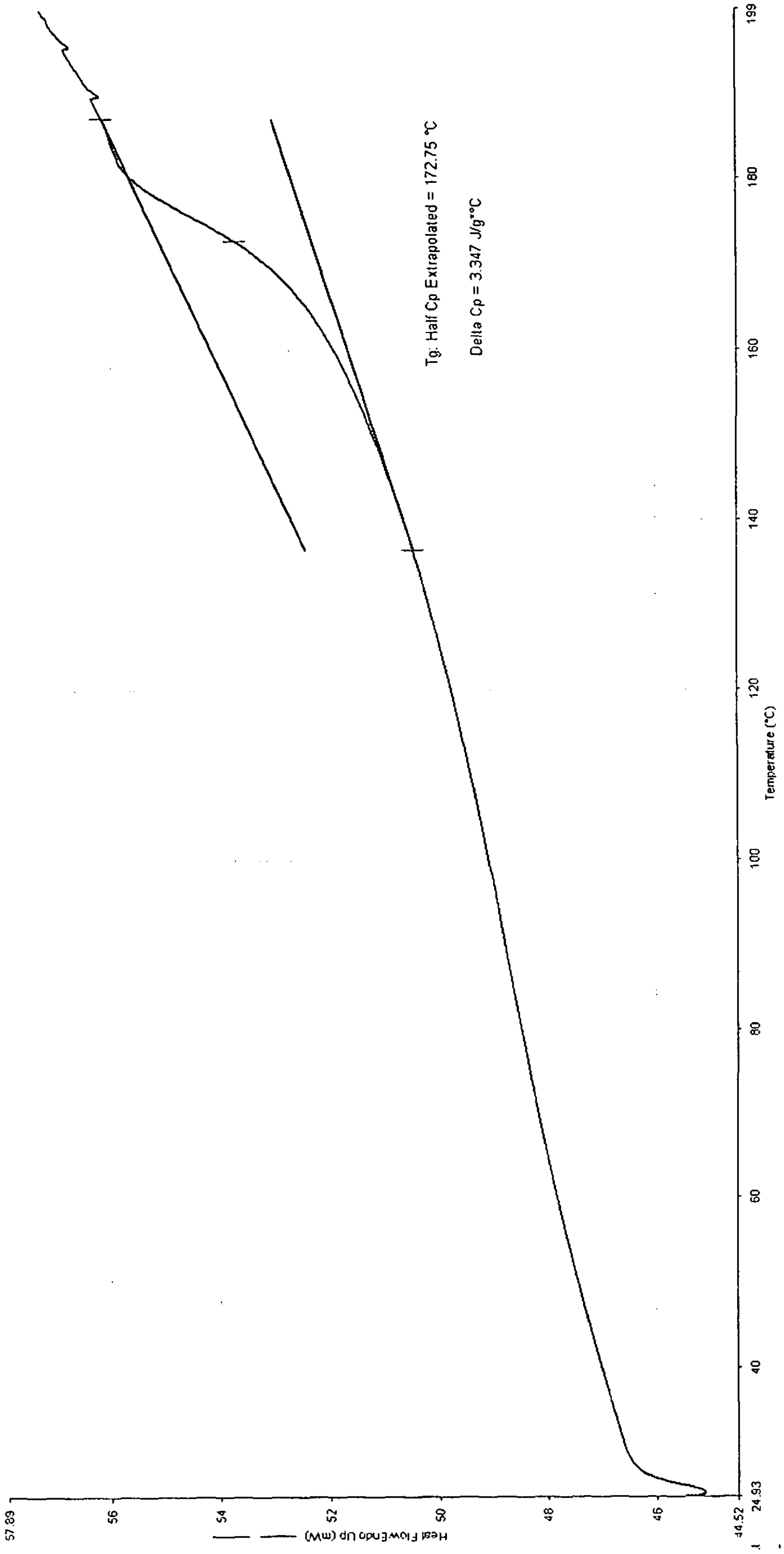
Tg: Half Cp Extrapolated = 172.75 °C
Delta Cp = 3.347 J/g*°C
Temperature (°C)
Heat Flow Endo Up (mW)
24.93
40
60
80
100
120
140
160
180
199
57.89
56
54
52
50
48
46
44.52

Figure 3

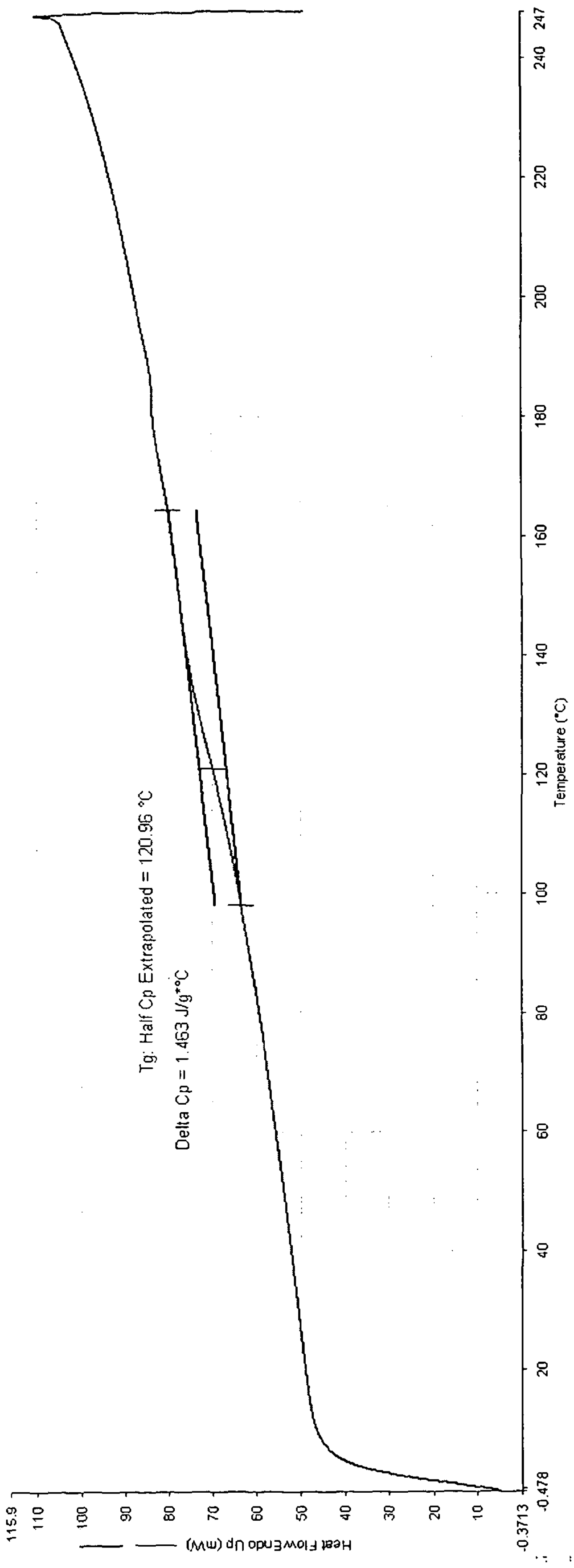
Tg: Half Cp Extrapolated = 120.96 °C
Delta Cp = 1.463 J/g*°C
Heat FlowEndo Up (mW)
115.9
110
100
90
80
70
60
50
40
30
20
10
-0.3713
-0.478
20
40
60
80
100
120
140
160
180
200
220
240
247
Temperature (°C)

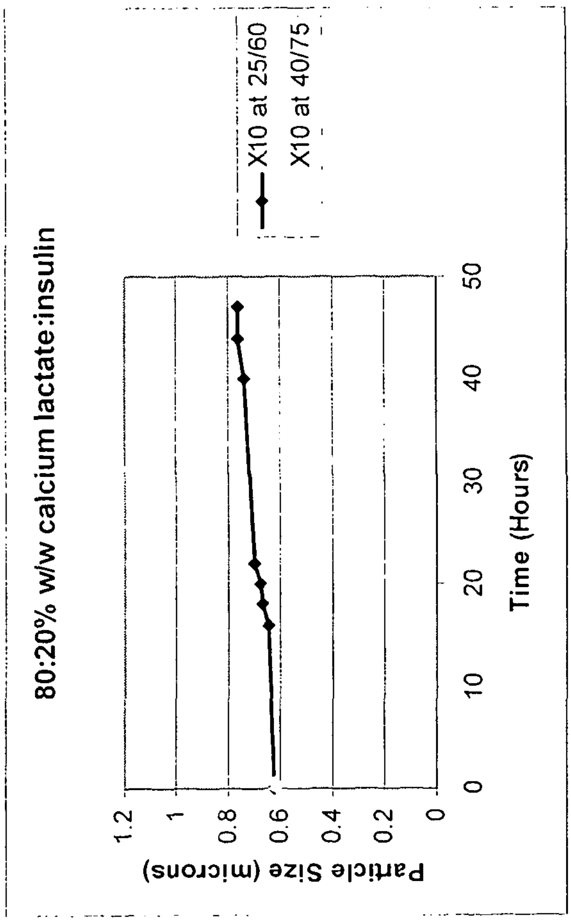
80:20% w/w calcium lactate:insulin
Particle Size (microns)
1.2
1
0.8
0.6
0.4
0.2
0
0
10
20
30
40
50
Time (Hours)
X10 at 25/60
X10 at 40/75

Figure 4

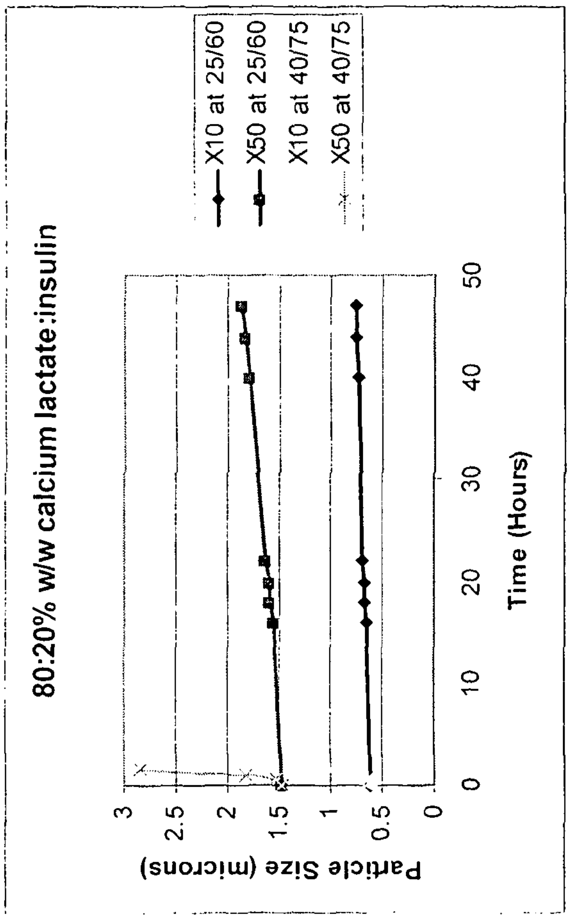
80:20% w/w calcium lactate:insulin
Particle Size (microns)
3
2.5
2
1.5
1
0.5
0
0
10
20
30
40
50
Time (Hours)
X10 at 25/60
X50 at 25/60
X10 at 40/75
X50 at 40/75

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1131059 A, Jago
- US 6528096 B, Chiesi
- WO 962348 A
- WO 9603978 A
- WO 9011756 A
- WO 9524183 A
- WO 9816205 A
- EP 0563455 A
- WO 2007042822 A
- WO 9947174 A
- WO 8906976 A
- WO 0018259 A
- WO 2004060343 A
- WO 03079993 A
- WO 9218164 A
- WO 9615814 A
- WO 9640077 A
- WO 9817257 A
- WO 0232899 A
- WO 0232898 A
- WO 03064419 A
- WO 03064418 A
- WO 03064417 A
- WO 0230390 A
- WO 03017970 A
- WO 03017979 A


### Non-patent literature cited in the description


- *Pharm Res.,* April 1998, vol. 15 (4), 562-9
- **ZENG.** *J Pharm Sci.,* September 2001, vol. 90 (9), 1424-34, http://www3.interscience.wiley.com/cgi-bin/abstract/107613204/ABSTRACT
- **MAA.** *Pharmaceutical Research,* 1999, vol. 16 (2
- **R.L. CARR.** *Chem Eng.,* 1965, vol. 72, 163-168